# EUROPEAN PATENT APPLICATION

(11) **EP 4 303 303 A1**
(43) Date of publication of application: **10.01.2024**
(21) Application number: 22763402.9
(22) Date of filing: 03.03.2022
(51) Int. Cl.: C12N 15/11, A61K 31/712, A61K 31/7125, A61P 43/00, C07F 9/09

(54) **ACYCLIC THREONINOL NUCLEIC ACID**

(30) Priority: 03.03.2021 JP 2021033170
(71) Applicant: The University of Tokyo, Bunkyo-ku, Tokyo 113-8654 (JP); AGC INC., Chiyoda-ku, Tokyo 1008405 (JP)
(72) Inventor: OKAZOE Takashi, Tokyo 100-8405 (JP); AIKAWA Kohsuke, Tokyo 113-8654 (JP); WATANABE Honoka, Tokyo 113-8654 (JP); OKAMOTO Akimitsu, Tokyo 113-8654 (JP); MORIHIRO Kunihiko, Tokyo 113-8654 (JP); KAGEYAMA Taiichi, Tokyo 113-8654 (JP)
(74) Representative: TBK
(86) International application number: PCT/JP2022/009209
(87) International publication number: WO 2022/186350

(57) **Abstract**

The present invention provides a nucleic acid having excellent cell membrane permeability.

The present invention relates to: a nucleic acid having a structure represented by the following general formula (A1) or (A2) [in the formula, R⁰ is an alkyl group having 1 to 30 carbon atoms that is substituted with one or more fluorine atoms, a group having 1 to 5 etheric oxygen atoms between carbon atoms of an alkyl group having 2 to 30 carbon atoms that is substituted with one or more fluorine atoms, an alkyl group having 10 to 30 carbon atoms that is not substituted with a fluorine atom, or a group having 1 to 5 etheric oxygen atoms between carbon atoms of an alkyl group having 10 to 30 carbon atoms that is not substituted with a fluorine atom; n11 and n12 each independently represent an integer of 1 or more; B is a nucleobase; and a filled circle indicates a bond]; a cell membrane permeabilizing agent containing the nucleic acid as an active ingredient; and a nucleic acid drug containing the nucleic acid as an active ingredient.

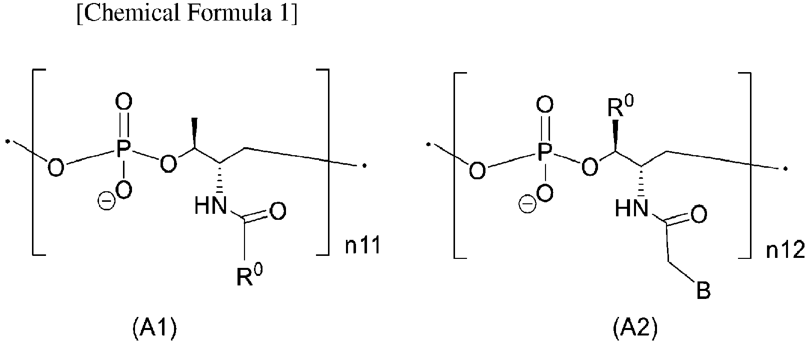

## Description

### [Technical Field]

The present invention relates to an acyclic threoninol-type nucleic acid excellent in cell membrane permeability.

Priority is claimed on Japanese Patent Application No. 2021-033170, filed on March 3, 2021, the content of which is incorporated herein by reference.

### [Background Art]

Antibody drugs are excellent as therapeutic agents for cancer and intractable diseases, because they can be formulated into drugs so long as antibodies can be produced by using the immune system, even against proteins that cannot be targeted by low molecular weight drugs. Further, antibody drugs have the advantages of being highly specific to target molecules and having few side effects. However, it is difficult for antibody drugs to pass through cell membranes and enter cells, and for this reason, it is difficult to use molecules other than those on the cell surface as target molecules.

Nucleic acid drugs using oligonucleotides are being studied as the next generation of drug discovery after antibody drugs. Nucleic acid drugs have the advantages of being highly specific to target molecules and having few side effects. However, like antibody drugs, nucleic acid drugs also have low cell membrane permeability, which makes it difficult for them to reach the target molecules present in the cells. In particular, since siRNA is double-stranded, its molecular weight and negative charge are both larger than those of antisense RNA, and its cell membrane permeability is lower than that of antisense RNA, thus requiring drug delivery by a carrier. As drug delivery agents, those using lipid nanoparticles (Patent Document 1) and those using cationic polymer nanoparticles (Patent Document 2) are known. However, there are many things that need to be improved in view of the efficiency of cell membrane permeability and toxicity concerns.

For example, compounds having polyfluorinated structures are known to be stable in vivo, have low toxicity and are excellent for cellular uptake and escape from endosomes (Non-Patent Document 1). It has been reported that by taking advantage of this property, a peptide dendrimer using lysine in which the amino group on the side chain is perfluoroacylated as a constituent amino acid can be used for gene delivery (Non-Patent Document 2). Further, the introduction of polyfluorinated structures into oligonucleotides or peptide nucleic acids as cell membrane-permeable moieties has also been investigated (Patent Document 3, and Non-Patent Documents 3 to 6).

On the other hand, since the phosphodiester bonds of nucleic acids are susceptible to degradation by nucleases, nucleic acid drugs also have the issue of stability when administered to living organisms. This is because if the stability is low, they will be degraded in vivo before reaching the target tissue, and the intended drug efficacy cannot be obtained. In order to increase the stability of nucleic acids, chimeric nucleic acids with artificial nucleic acids are used which are superior in nuclease resistance and the like to natural nucleic acids. Examples of the artificial nucleic acid include acyclic glycol nucleic acid (GNA), peptide nucleic acid (PNA), acyclic threoninol nucleic acid (aTNA), and serinol nucleic acid (SNA) (Non-Patent Document 7).

### [Citation List]

### [Patent Document]

[Patent Document 1] International Patent Publication No. 2011/036557
[Patent Document 2] International Patent Publication No. 2017/212006
[Patent Document 3] International Patent Publication No. 2012/130941
[Patent Document 4] Japanese Unexamined Patent Application, First Publication No. 2006-321797
[Patent Document 5] International Patent Publication No. 2000/056694

### [Non-Patent Documents]

[Non-Patent Document 1] Zhang et al., MRS Communications, 2018, vol. 8, p. 303-313.
[Non-Patent Document 2] Cai et al., ACS Applied Materials and Interfaces, 2016, vol. 8, p. 5821-5832.
[Non-Patent Document 3] Godeau et al., Medicinal Chemistry Communications, 2010, vol. 1, p. 76-78.
[Non-Patent Document 4] Ellipilli et al., Chemical Communications, 2016, vol. 52, p. 521-524.
[Non-Patent Document 5] Rochambeaua et al., Polymer Chemistry, 2016, vol. 7, p. 4998-5003.
[Non-Patent Document 6] Metelev et al., Theranostics, 2017, vol. 7, p. 3354-3368.
[Non-Patent Document 7] Murayama et al., Chemistry A European Journal, 2013, vol. 19, p. 14151-14158.

### [Summary of Invention]

### [Technical Problem]

The present invention has an object of providing a nucleic acid excellent in cell membrane permeability.

### [Solution to Problem]

The inventors of the present invention have found that the introduction of an alkyl group which may be substituted with a fluorine atom into the side chain of an acyclic threoninol-type nucleic acid (aTNA-type nucleic acid) improves cell membrane permeability, thereby completing the present invention.

That is, the present invention is as follows.
[1] A nucleic acid having a structure represented by: the following general formula (A1) or (A2), 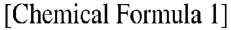 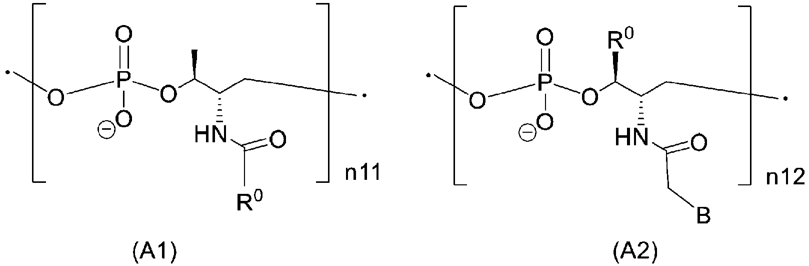 [in the formula, R⁰ is an alkyl group having 1 to 30 carbon atoms that is substituted with one or more fluorine atoms, a group having 1 to 5 etheric oxygen atoms between carbon atoms of an alkyl group having 2 to 30 carbon atoms that is substituted with one or more fluorine atoms, an alkyl group having 10 to 30 carbon atoms not substituted by fluorine atoms, or a group having 1 to 5 etheric oxygen atoms between carbon atoms of an alkyl group having 10 to 30 carbon atoms not substituted by fluorine atoms; n11 and n12 each independently represent an integer of 1 or more; B is a nucleobase; and a filled circle indicates a bond].
[2] The nucleic acid according to [1] above, wherein the aforementioned R⁰ is an alkyl group having 1 to 30 carbon atoms that is substituted with at least two fluorine atoms.
[3] The nucleic acid according to [2] above, wherein the aforementioned R⁰ is a perfluoroalkyl group having 1 to 10 carbon atoms, or a group having 1 to 5 etheric oxygen atoms between carbon atoms of a perfluoroalkyl group having 1 to 10 carbon atoms.
[4] The nucleic acid according to [1] above, wherein the aforementioned R⁰ is an alkyl group having 10 to 30 carbon atoms that is not substituted with a fluorine atom, or a group having 1 to 5 etheric oxygen atoms between carbon atoms of an alkyl group having 10 to 30 carbon atoms that is not substituted with a fluorine atom.
[5] The nucleic acid according to any one of [1] to [4] above, wherein n11 or n12 is 5 or more.
[6] The nucleic acid according to any one of [1] to [5] above, which is cell membrane permeable.
[7] A cell membrane permeabilizing agent containing the nucleic acid according to any one of [1] to [6] above as an active ingredient.
[8] A nucleic acid drug containing the nucleic acid according to any one of [1] to [6] above as an active ingredient.

### [Advantageous Effects of Invention]

The nucleic acid according to the present invention is excellent in cell membrane permeability because an alkyl group has been introduced into the side chain of an aTNA-type nucleic acid. For this reason, this nucleic acid is expected to be used in the pharmaceutical field as a physiologically active substance.

### [Brief Description of Drawings]

FIG. 1 is a diagram showing the results of flow cytometry of cells having each fluorescein-labeled nucleic acid introduced in Example 1.
FIG. 2 is a diagram showing the results of flow cytometry of cells having each fluorescein-labeled nucleic acid introduced in Example 2.
FIG. 3 is a diagram showing the results of flow cytometry of cells having each fluorescein-labeled nucleic acid introduced in Example 3.
FIG. 4 is a diagram showing the results of flow cytometry of cells having each fluorescein-labeled nucleic acid introduced in Example 4.
FIG. 5 is a diagram showing the results of flow cytometry of cells having each fluorescein-labeled nucleic acid introduced in Example 5.
FIG. 6 is a diagram showing the results of flow cytometry of cells having each fluorescein-labeled nucleic acid introduced in Example 6.

### [Description of Embodiments]

In the present invention and the specification of the present application, the term "nucleic acid" means a molecule in which nucleotides are bound by a phosphodiester bond. The nucleotides include not only natural nucleotides (naturally occurring nucleotides) such as DNA and RNA, but also artificial nucleotides that are obtained by modifying natural nucleotides and can be bonded to natural nucleotides by a phosphodiester bond. Examples of the artificial nucleotides include those in which the side chain or the like of a natural nucleotide has been modified with a functional group such as an amino group, those in which the hydroxyl group at the 2'-position of the ribose backbone has been substituted with a methoxy group, a fluoro group, a methoxyethyl group or the like, phosphorothioate-type nucleotides (those in which the oxygen atom of the phosphate group has been substituted with a sulfur atom), morpholino-type nucleotides (those in which ribose and deoxyribose have been substituted with a morpholine ring), bridged nucleic acids (BNA), hexitol nucleic acids (HNA), locked nucleic acids (LNA), peptide nucleic acids (PNA), threose nucleic acids (TNA), glycerol nucleic acids (GNA), and cyclohexenyl nucleic acids (CeNA). Further, the term "nucleic acid" includes all of the following: a molecule in which only one or more natural nucleotides, such as DNA or RNA, are bound by a phosphodiester bond; a molecule in which one or more natural nucleotides and one or more artificial nucleotides are bound by a phosphodiester bond; and a molecule in which only one or more artificial nucleotides are bound by a phosphodiester bond.

In the present invention and the specification of the present application, "Cₚ₁₋ₚ₂" (p1 and p2 are positive integers satisfying a relationship of p1 < p2) means a group in which the number of carbon atoms is from p1 to p2.

In the present invention and the specification of the present application, a "C₁₋₃₀ alkyl group" is an alkyl group having 1 to 30 carbon atoms, and may be linear or branched. A "C₂₋₃₀ alkyl group" is an alkyl group having 2 to 30 carbon atoms and may be linear or branched. Examples of the C₁₋₃₀ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group, an octacosyl group, a nonacosyl group, and a triacontyl group.

In the present invention and the specification of the present application, a "C₁₋₂₀ alkyl group" is an alkyl group having 1 to 20 carbon atoms, and may be linear or branched. A "C₂₋₂₀ alkyl group" is an alkyl group having 2 to 20 carbon atoms and may be linear or branched. Examples of the C₁₋₂₀ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an eicosyl group.

In the present invention and the specification of the present application, a "C₁₋₁₀ alkyl group" is an alkyl group having 1 to 10 carbon atoms, and may be linear or branched. A "C₂₋₁₀ alkyl group" is an alkyl group having 2 to 10 carbon atoms and may be linear or branched. Examples of the C₁₋₁₀ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, and a decyl group.

In the present invention and the specification of the present application, a "₁₀₋₃₀ alkyl group" is an alkyl group having 10 to 30 carbon atoms, and may be linear or branched. Examples of the C₁₀₋₃₀ alkyl group include an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group, an octacosyl group, a nonacosyl group, and a triacontyl group.

In the present invention and the specification of the present application, a "C₁₋₆ alkyl group" is an alkyl group having 1 to 6 carbon atoms, and may be linear or branched. Examples of the C₁₋₆ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, and a hexyl group.

In the present invention and the specification of the present application, an "alkylene group" is a divalent group obtained by removing two hydrogen atoms from a saturated hydrocarbon, and may be linear or branched. Examples of the alkylene group include a methylene group, an ethylene group, a trimethylene group, a tetramethylene group, a pentamethylene group, a hexamethylene group, a heptamethylene group, an octamethylene group, a nonamethylene group, a methylmethylene group, an ethylmethylene group, a methylethylene group, a methylpropylene group, an ethylethylene group, a dimethylmethylene group, a 1,2-dimethylethylene group, a 1,1-dimethylethylene group, a 1-ethylpropylene group, a 2-ethylpropylene group, a 1,2-dimethylpropylene group, a 2,2-dimethylpropylene group, a 1-propylpropylene group, a 2-propylpropylene group, a 1-methyl-1-ethylpropylene group, a 1-methyl-2-ethylpropylene group, a 1-ethyl-2-methyl-propylene group, a 2-methyl-2-ethyl-propylene group, a 1-methylbutylene group, a 2-methylbutylene group, a 3-methylbutylene group, a 2-ethylbutylene group, a 1-methylpentylene group, a 2-ethylpentylene group, and a 1-methylhexylene group.

In the present invention and the specification of the present application, a "C₁₋₂₀ perfluoroalkyl group" is a group in which all hydrogen atoms of an alkyl group having 1 to 20 carbon atoms have been substituted with fluorine atoms. Examples of the C₁₋₁₀ perfluoroalkyl group include a perfluoromethyl group, a perfluoroethyl group, a perfluoropropyl group, a perfluoroisopropyl group, a perfluorobutyl group, a perfluoroisobutyl group, a perfluorosec-butyl group, a perfluorotert-butyl group, a perfluoropentyl group, a perfluoroisopentyl group, a perfluoroneopentyl group, a perfluorotert-pentyl group, a perfluorohexyl group, a perfluoroheptyl group, a perfluorooctyl group, a perfluorononyl group, a perfluorodecyl group, a perfluoroundecyl group, a perfluorododecyl group, a perfluorotridecyl group, a perfluorotetradecyl group, a perfluoropentadecyl group, a perfluorohexadecyl group, a perfluoroheptadecyl group, a perfluorooctadecyl group, a perfluorononadecyl group, and a perfluoroeicosyl group.

In the present invention and the specification of the present application, a "perfluoroalkylene group" is a group in which all hydrogen atoms of an alkylene group have been substituted with fluorine atoms. Examples of the perfluoroalkylene group include groups in which all hydrogen atoms of the above-mentioned alkylene groups have been substituted with fluorine atoms.

In the present invention and the specification of the present application, an "etheric oxygen atom" is an oxygen atom that connects between carbon atoms, and does not include an oxygen atom in which oxygen atoms are connected to each other in series. An alkyl group having Nc carbon atoms (Nc is an integer of 2 or more) can have up to Nc-1 etheric oxygen atoms. Further, a "C₂₋₁₀ alkyl group having an etheric oxygen atom between carbon atoms" is a group having at least one etheric oxygen atom between carbon atoms of the C₂₋₁₀ alkyl group. Hereinafter, an alkyl group having an etheric oxygen atom may be referred to as an "ether bond-containing alkyl group".

In the present invention and the specification of the present application, a "C₂₋₁₀ perfluoroalkyl group having an etheric oxygen atom between carbon atoms" is a group in which all hydrogen atoms of an ether bond-containing C₂₋₁₀ alkyl group having at least one etheric oxygen atom between carbon atoms of the C₂₋₁₀ alkyl group have been substituted with fluorine atoms. Hereinafter, a perfluoroalkyl group having an etheric oxygen atom may be referred to as an "ether bond-containing perfluoroalkyl group".

In the present invention and the specification of the present application, a "halogen atom" refers to a fluorine atom, a chlorine atom, a bromine atom, or an iodine atom. A "halogen atom other than a fluorine atom" refers to a chlorine atom, a bromine atom, or an iodine atom. As an example of the "halogen atom other than a fluorine atom", a chlorine atom or a bromine atom is preferred, and a chlorine atom is particularly preferred.

Further, hereinafter, a "compound (X)" refers to a compound represented by a formula (X).

### <Nucleic acid>

The nucleic acid according to the present invention has an alkyl group, which may be substituted with a fluorine atom, at a specific site of an aTNA-type nucleic acid. The nucleic acid, like other artificial nucleic acids, is expected to be used in the pharmaceutical field as a physiologically active substance. More specifically, the nucleic acid according to the present invention has a structure represented by the following general formula (A1) or (A2). It should be noted that hereinafter, the "structure represented by the general formula (A1)" may be referred to as a "structure (A1)", and the "structure represented by the general formula (A2)" may be referred to as a "structure (A2)".

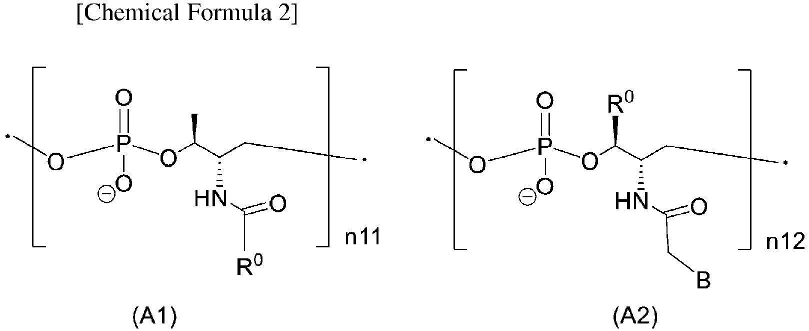

In the general formulas (A1) and (A2), R⁰ is a C₁₋₃₀ alkyl group substituted with at least one fluorine atom (C₁₋₃₀ fluoroalkyl group), or a C₁₀₋₃₀ alkyl group that is not substituted with a fluorine atom. When the number of carbon atoms in this alkyl group is 2 or more, this alkyl group may have 1 to 5 etheric oxygen atoms between carbon atoms. In the present invention and the specification of the present application, a "C₁₋₃₀ fluoroalkyl group (when the number of carbon atoms in this alkyl group is 2 or more, this alkyl group may have 1 to 5 etheric oxygen atoms between carbon atoms)" means "a C₁₋₃₀ fluoroalkyl group, or a group having 1 to 5 etheric oxygen atoms between carbon atoms of a C₂₋₃₀ fluoroalkyl group".

When R⁰ is a C₁₋₃₀ fluoroalkyl group, one or more hydrogen atoms bonded to carbon atoms may be further substituted with halogen atoms other than fluorine atoms. For the structure (A1) or structure (A2), R⁰ is preferably a C₁₋₃₀ fluoroalkyl group substituted with at least two fluorine atoms. In particular, R⁰ is preferably a C₁₋₂₀ fluoroalkyl group, more preferably a C₁₋₁₅ fluoroalkyl group, still more preferably a C₂₋₁₅ fluoroalkyl group, and even more preferably a C₆₋₁₀ fluoroalkyl group. When R⁰ is a C₁₋₃₀ fluoroalkyl group, the number of hydrogen atoms substituted with fluorine atoms is not particularly limited as long as it is 1 or more, and for example, it is preferably 3 or more, more preferably 6 or more, and still more preferably 7 or more.

More specifically, when R⁰ is a C₁₋₃₀ fluoroalkyl group, R⁰ is preferably a group represented by the following general formula (f-1) or (f-2). Here, Rf^{P} is a fully halogenated C₁₋₂₀ alkyl group (a group in which all hydrogen atoms of the C₁₋₂₀ alkyl group have been substituted with halogen atoms), which has one or more fluorine atoms. When Rf^{P} has 2 or more carbon atoms, that is, when it is a fully halogenated C₂₋₂₀ alkyl group, it may have 1 to 5 etheric oxygen atoms between carbon atoms. In the present invention and the specification of the present application, a "fully halogenated C₂₋₂₀ alkyl group which may have 1 to 5 etheric oxygen atoms between carbon atoms" refers to "a fully halogenated C₂₋₂₀ alkyl group or a group having 1 to 5 etheric oxygen atoms between the carbon atoms of a fully halogenated C₂₋₂₀ alkyl group".

In the general formula (f-2), two Rf^{P} groups may be groups of the same type or groups of different types with each other. Rf' is preferably a C₁₋₂₀ perfluoroalkyl group (a group in which all hydrogen atoms of a C₁₋₂₀ alkyl group have been substituted with fluorine atoms).

In the following general formula (f-1) or (f-2), n1 is an integer of 0 to 10, and n2 is an integer of 0 to 9. When n1 and n2 are 0, both represent single bonds. That is, when n1 is 0, the group represented by the general formula (f-1) is -Rf^{P}, and when n2 is 0, the group represented by the general formula (f-2) is -CH(Rf^{P})₂.

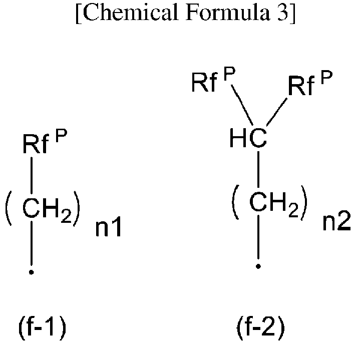

When R⁰ is a group represented by the general formula (f-1), R⁰ is: preferably a group in which Rf^{P} is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n1 is an integer of 0 to 4; more preferably a group in which Rf^{P} is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n1 is an integer of 0 to 2; still more preferably a group in which Rf^{P} is a trifluoromethyl group, a pentafluoroethyl group, a heptafluoropropyl group, a nonafluorobutyl group, a perfluoropentyl group, or a perfluorohexyl group, and n1 is an integer of 0 to 2 (provided that a group in which n1 is 1 and Rf^{P} is a trifluoromethyl group is excluded); and even more preferably a group in which Rf^{P} is a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n1 is 0.

When R⁰ is a group represented by the general formula (f-2), R⁰ is: preferably a group in which Rf' is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n2 is an integer of 0 to 4; more preferably a group in which Rf^{P} is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n2 is an integer of 0 to 2; still more preferably a group in which Rf is a trifluoromethyl group, pentafluoroethyl group, heptafluoropropyl group, nonafluorobutyl group, perfluoropentyl group, or perfluorohexyl group, and n2 is an integer of 0 to 2 (provided that a group in which n2 is 0 or 1 and Rf^{P} is a trifluoromethyl group is excluded); and even more preferably a group in which Rf^{P} is a pentafluoroethyl group, a heptafluoropropyl group, a nonafluorobutyl group, a perfluoropentyl group, or a perfluorohexyl group, and n2 is 0.

When R⁰ is a C₁₋₃₀ fluoroalkyl group, examples of R⁰ include a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, a perfluorodecyl group, a difluoromethyl group, a 1,1-difluoroethyl group, a 2,2-difluoroethyl group, a 1,1,2,2-tetrafluoroethyl group, a 1,1,2,2,3,3-hexafluoropropyl group, a 1,1,2,3,3,3-hexafluoropropyl group, a 1,1,2,2,3,3-hexafluorohexyl group, a 1,1,2,2,3,3-hexafluorooctyl group, a 1,1,2,2,3,3-hexafluorodecyl group, a 1,1,2,2,3,3-hexafluorooctadecyl group, and a 1,1,2,2,3,3-hexafluorohexacosyl group. The nucleic acid according to the present invention is preferably a nucleic acid having a structure in which R⁰ is a C₁₋₃₀ perfluoroalkyl group in the general formula (A1) or (A2), more preferably a nucleic acid having a structure in which R⁰ is a C₁₋₂₀ perfluoroalkyl group in the general formula (A1) or (A2), and still more preferably a nucleic acid having a structure in which R⁰ is a C₁₋₁₀ perfluoroalkyl group in the general formula (A1) or (A2).

When R⁰ is a C₁₀₋₃₀ alkyl group, for the structure (A1) or structure (A2), R⁰ is preferably a C₁₀₋₂₅ alkyl group, more preferably a C₁₅₋₂₅ alkyl group, and still more preferably a C₁₅₋₂₃ alkyl group. Like a fluoroalkyl group, a sufficiently long alkyl group is highly hydrophobic and contributes to the cell membrane permeability of nucleic acids having the structure (A1) or structure (A2).

In the general formula (A2), B is a nucleobase. As the nucleobase, a nucleobase included in a natural nucleic acid, a nucleobase having a structure similar to that of a natural nucleobase, and a modified base obtained by subjecting these bases to various modifications are preferred. Examples of modifications of bases include alkylation, hydroxylation, alkoxylation, acylation, dihydroxylation, amination, formylation, and halogenation. Examples of the nucleobase having a structure similar to that of a natural nucleobase include triazole, imidazole, azapyrimidine, and azapurine. Specific examples of the nucleobase represented by B include adenine, guanine, cytosine, thymine, uracil, 1-methyladenine, N6-methyladenine, 7-methylguanine, 5-methylcytosine, 1-methylthymine, 5-methyluracil, 5-hydroxymethylcytosine, 5-hydroxyuracil, 5-hydroxymethyluracil, dihydrouracil, dihydrothymine, dihydrocytosine, 2,6-diaminoadenine, 2,6-diaminoguanine, 6-thioguanine, 2-thioadenine, 2-thiocytosine, 4-thiouracil, 5-fluorouracil, 5-iodouracil, 5-halogenocytosine, 5-fluorocytosine, 5-trihalogenomethyluracil, 5-trifluoromethyluracil, 5-azathymine, 5-azacytosine, 6-azauracil, 8-azaadenine, 7-deazaadenine, 7-deazaguanine, and 3-deazauracil. The nucleic acid according to the present invention is preferably a nucleic acid having a structure in which B is adenine, guanine, cytosine, thymine, or uracil in the general formula (A2).

In the general formulas (A1) and (A2), n11 and n12 each independently represents an integer of 1 or more. n11 and n12 are the number of repetitions per molecule of each structure, and the larger the number, the higher the hydrophobicity. As a result, the cell membrane permeability of the nucleic acid is further improved. With regard to the nucleic acid according to the present invention, n11 and n12 are preferably 2 or more, and more preferably 5 or more. Further, n11 and n12 are preferably 10 or less, more preferably 8 or less, and still more preferably 6 or less, because structures similar to those of natural double-stranded nucleic acids and single-stranded nucleic acids are likely to be taken. When n11 and n12 are 2 or more, a plurality of structures (A1) or structures (A2) may be the same structure or different structures from each other.

In the general formulas (A1) and (A2), a filled circle indicates a bond. The nucleic acid according to the present invention may be a nucleic acid having the structure (A1) or structure (A2), and the position where the structure (A1) or structure (A2) is introduced is not particularly limited, and it may be introduced into any site as long as it does not impair the function of the nucleic acid. For example, the structure (A1) or structure (A2) may be bonded directly or indirectly to the 5'-end or 3'-end of the nucleic acid, or may be introduced between two nucleotides.

Among the nucleic acids according to the present invention, it is preferable that in a nucleic acid having the structure (A1) or structure (A2) at the 5'-end of the nucleic acid, a bond extending from the carbon atom at the end of the structure (A1) or structure (A2) is bound to a hydroxy group, and a bond extending from the oxygen atom of the phosphate group at the end of the structure (A1) or structure (A2) is bound to the sugar at the 5'-end of the nucleic acid. Among the nucleic acids according to the present invention, it is preferable that in a nucleic acid having the structure (A1) or structure (A2) at the 3'-end of the nucleic acid, a bond extending from the carbon atom at the end of the structure (A1) or structure (A2) is bound to the phosphate group at the 3'-end of the nucleic acid, and a bond extending from the oxygen atom of the phosphate group at the end of the structure (A1) or structure (A2) is bound to a hydrogen atom.

Among the nucleic acids according to the present invention, it is preferable that in a nucleic acid in which the structure (A1) or structure (A2) has been introduced between two nucleotides, since it is structurally more similar to natural nucleic acids, a bond extending from the carbon atom at the end of the structure (A1) or structure (A2) is bound to the phosphate group of another nucleotide, and a bond extending from the oxygen atom of the phosphate group at the end of the structure (A1) or structure (A2) is bound to the sugar of another nucleotide.

In the nucleic acid according to the present invention, when a highly hydrophobic R⁰ group is introduced into an aTNA-type nucleic acid, because it is introduced as the structure (A1) or structure (A2), for example, when a DNA double helix structure is formed, the R⁰ group is exposed outside the helical structure, making it possible to form a more stable double helix structure, and the R⁰ group exposed on the surface can improve the cell membrane permeability. That is, the nucleic acid according to the present invention is useful as a cell membrane permeabilizing agent. In addition, since the nucleic acid according to the present invention does not contain a nitrogen atom in a chain composed of the phosphate group of the nucleic acid and the phosphodiester bond of the sugar, for example, an intermediate is relatively stable, and is also easy to synthesize, when synthesized by a phosphoramidite method.

In the nucleic acid according to the present invention, the nucleic acid into which the structure (A1) or structure (A2) is introduced is not particularly limited, and may be a nucleic acid in which all the nucleotides contained are natural nucleotides, or may be a nucleic acid in which some or all of the nucleotides are artificial nucleotides. Further, it may be a single-stranded nucleic acid or a double-stranded nucleic acid. Examples of such nucleic acids include genomic DNA, cDNA, mRNA, microRNA, siRNA, antisense oligonucleotides, nucleic acid aptamers, decoy nucleic acids, and CpG (cytosine-phosphate-guanine) oligonucleotides. Further, it may also be an expression vector that expresses a target gene or siRNA in cells.

In the nucleic acid according to the present invention, the structure (A1) or structure (A2) can be introduced by various coupling reactions into the target nucleic acid into which the structure is to be introduced. For example, the structure (A1) or structure (A2) can be easily introduced into the nucleic acid by performing the phosphoramidite method using a phosphoramidite containing the structure (A1) or structure (A2) as a raw material. A widely used automated nucleic acid synthesizer utilizes the phosphoramidite method. Therefore, by using a phosphoramidite containing the structure (A1) or structure (A2) as a raw material, nucleic acids in which the structure (A1) or structure (A2) has been introduced at the desired positions, with respect to nucleic acids of various base sequences, can be easily synthesized by the automated synthesizer.

Examples of the phosphoramidite containing the structure (A1) or structure (A2) include, among phosphoramidites generally used for nucleic acid synthesis, a compound in which the sugar and the phosphate group are linked via the structure (A1) and a compound in which the nucleoside moiety is substituted with an organic group containing the structure (A2).

The nucleic acid according to the present invention may be a nucleic acid having only the structure (A1) or structure (A2). In this case, the bonds at both ends of the structure (A1) or structure (A2) are bonded to hydrogen atoms or hydroxy groups.

The synthesized nucleic acid of interest can be isolated and purified, for example, by various methods such as ion chromatography, gel filtration chromatography, reverse phase chromatography, and normal phase chromatography.

The nucleic acid according to the present invention may be subjected to various modifications as long as the functions of the nucleic acid into which the structure (A1) or structure (A2) is introduced are not impaired and the effects of the present invention are not impaired. Examples of such modifications include glycosylations, lipid modifications, and peptide modifications.

The R⁰ group is highly hydrophobic and has a high affinity for cell membranes. For this reason, the nucleic acid according to the present invention into which the structure (A1) or structure (A2) containing the R⁰ group has been introduced has better cell membrane permeability than that of the nucleic acid into which the R⁰ group has not been introduced. By taking advantage of this property, the nucleic acid according to the present invention is particularly preferred as an active ingredient of a nucleic acid drug. For example, by introducing the structure (A1) or structure (A2) into a functional nucleic acid that exhibits some physiological activity, without impairing its function, by being taken up into a target cell in vivo, it is possible to improve the incorporation efficiency of the functional nucleic acid into the target cell. For example, by introducing the structure (A1) or structure (A2) into a nucleic acid that has pharmacological activity but cannot reach into target cells in vivo, it is possible to improve the incorporation efficiency of the nucleic acid into the target cells. That is, by using the nucleic acid according to the present invention, a drug delivery system for delivering nucleic acid drugs into cells can be easily constructed.

### [Examples]

Hereinafter, the present invention will be described with reference to Examples, but the present invention is not limited to these Examples.

An NMR apparatus used for the analysis in Examples and Comparative Examples was JNM-ECZ400S (400 MHz) manufactured by JEOL Ltd., tetramethylsilane was used for obtaining a reference value of 0 ppm in ¹H-NMR, and C₆F₆ was used for obtaining a reference value of -162 ppm in ¹⁹F-NMR.

### <DNA synthesis>

In subsequent experiments, DNA synthesis was performed on an NTS H-8 DNA/RNA Synthesizer (manufactured by Nihon Techno Service Co., Ltd.) using commercially available reagents, various phosphoramidites (acetonitrile solutions, 0.1 M) and 5-ethylthio-1H-tetrazole (acetonitrile solution, 0.25 M) as an activator.

The synthesis of 5'-aTNA-N[PFC8]-modified DNA was performed as follows. First, DNA synthesis (trityl-off) was performed on a 1,000 Å CPG solid support column (1 µmole scale). Then, under a nitrogen atmosphere, an aTNA-N[PFC8] amidite solution (0.1 M acetonitrile solution, 300 µL) and an activator solution (300 µL) were mixed in the presence of CPG using a syringe. After 5 minutes, the obtained mixed solution was collected from the column and the DNA strands were capped, oxidized and unblocked on a DNA synthesizer.

Deprotection of the synthesized 5'-aTNA-N[PFC8]-modified DNA was carried out as follows. First, the CPG solid-supported DNA (trityl-off) was treated with a 28% aqueous ammonium hydroxide solution at 50°C for 12 hours. Then, this crude product solution was separated from the solid support and concentrated at 30°C under reduced pressure. The obtained concentrate was filtered through a 0.45 µm centrifugal filter prior to HPLC purification. The obtained filtrate was filtered through a 0.45 µm centrifugal filter and then purified by HPLC. The obtained solution was quantified by absorbance at 260 nm.

The deprotected DNA was purified by HPLC. HPLC purification was carried out under the following conditions.

Solvent (filtered through a 0.45 µm centrifugal filter): 100 mM triethylammonium acetate (TEAA) buffer (pH 7.0) and acetonitrile (HPLC grade)
Elution gradient: 3 to 95% acetonitrile (40 minutes)
Column: COSMOSIL packed column "5C18-MS-II" (4.6 ID × 150 nm, manufactured by Nacalai Tesque, Inc.)
Samples to be tested for each analysis: a solution obtained by dissolving crude DNA in 20 to 50 µL of ultrapure water was injected.
Detection: performed using a diode array detector while monitoring the absorbance at 260 nm.

### <Cell culture>

In subsequent experiments, cell culture was performed as follows.

HeLa cells were cultured in Dulbecco's modified Eagle's medium (DMEM, manufactured by Thermo Fisher Scientific, Inc.) supplemented with 10% FBS and 0.5% penicillin/streptomycin in a humidified atmosphere (5 vol% CO₂) at 37°C. Cells for image analysis were cultured in a 35 mm glass bottom dish (IWAKI, manufactured by AGC Techno Glass Co., Ltd.).

### <Confocal microscopy>

In subsequent experiments, cells were observed with a confocal microscope as follows.

A fluorescein-conjugated nucleic acid (500 µM, 5 µL) was added to HeLa cells and incubated at 37°C for 3 hours. Then, hoechst 33342 (2 µg/mL, 0.5 mL) was added, as a nuclear stain, to the cells and further incubated for 1 hour. After incubation, the solution was removed from each dish, and DMEM (1 mL) was added after washing with PBS(-). Then, each dish was placed on a confocal laser scanning microscope and fluorescence images were acquired with an excitation wavelength of 488 nm and an emission filter of greater than 505 nm.

### <Flow cytometry>

In subsequent experiments, flow cytometry was performed as follows.

HeLa cells were seeded in a 12-well plate so as to achieve a density of 10⁵ cells/well and cultured. On the day after seeding, the medium in each well was changed to DMEM medium (1 mL) containing a fluorescein-conjugated nucleic acid (2.5 µM) and incubated for 4 or 24 hours. Then, after washing the cell layer in the well twice with PBS, the cells were detached by treatment with 0.05% (w/v) trypsin (200 µL) at 37°C for 5 minutes. The recovered cells were suspended in DMEM (600 µL). The cell suspension was separated by centrifugation process (400 × g, 3 minutes), and PBS/1% BSA (500 µL) was added thereto. The ratio of fluorescent cells in this cell suspension and the mean fluorescence intensity were analyzed with a flow cytometer ("guava easyCyte8", manufactured by Luminex Corporation).

### [Example 1]

A nucleic acid having a structure (A1) in which R⁰ was a perfluorooctyl group was synthesized, and the cell membrane permeability was examined.

A phosphoramidite (aTNA-N[PFC8]) having the structure (A1) in which R⁰ was a perfluorooctyl group was synthesized as follows.

### (1) Amidite condensation

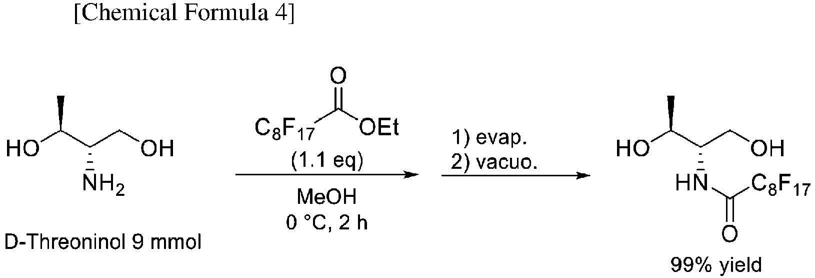

D-threoninol (0.95 g, 9 mmol) was dissolved in dry methanol (10 mL) in an ice bath, followed by dropwise addition of ethyl heptadecafluorononanoate (4.8 g, 9.9 mmol, in 5 mL of methanol solution). After stirring for 2 hours at 0°C, the solvent was removed by evaporation to obtain 4.9 g (yield: 99%) of the desired intermediate (N-(1,3-dihydroxybutan-2-yl)nonanamide).

¹H NMR (400 MHz, CDCl₃) δ 7.81 (d, J = 6.9 Hz, 1H), δ 4.14-4.07 (m, 1H), δ 4.02 (t, J = 5.5 Hz, 1H), δ 3.95-3.88 (m, 1H), δ 3.76-3.64 (m, 2H)
¹⁹F NMR (376 MHz, CDCl₃) δ -81.6 (s, 3F), δ -119.6 (s, 2F), δ -122.0 (s, 2F), δ -122.4 (s, 2F), δ -112.8 (s, 2F), δ -123.4 (s, 2F), δ -126.8 (s, 2F)

### (2) DMTr protection

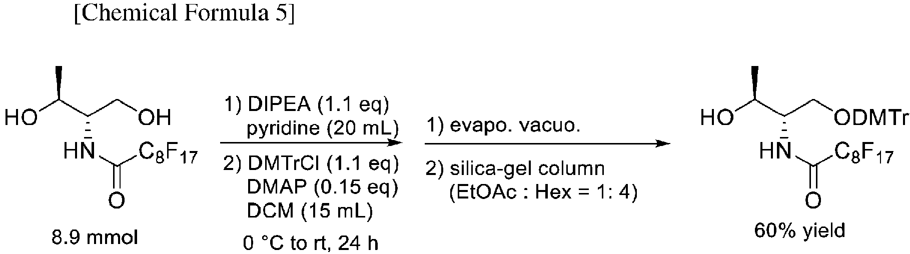

A dry pyridine solution (20 mL) containing the intermediate (N-(1,3-dihydroxybutan-2-yl)nonanamide) (1.8 g, 8.8 mmol) and diisopropylethylamine (DIPEA) (1.7 mL, 1.4 g, 10.5 mmol) was cooled on ice under nitrogen. Then, a dry dichloromethane solution (15 mL) containing 4,4'-dimethoxytrityl chloride (3.6 g, 10.5 mmol) and 4-dimethylaminopyridine (0.16 g, 1.3 mmol) was added to this mixture. After stirring the mixture at room temperature for 2.5 hours, the solvent was removed, followed by silica gel column chromatography (hexane: ethyl acetate: Et3N = 80:20:3 (volume ratio)) to obtain 3.6 g (7.2 mmol) of a DMTr-protected intermediate (yield: 60%).

¹H NMR (400 MHz, CDCl₃) δ 7.36-7.20 (m, 8H), δ 7.06 (d, J = 8.7 Hz, 1H), δ 3.78 (s, 6H), δ 3.48 (dd, J = 4.2 Hz, 9.6 Hz, 1H), δ 3.28 (dd, J = 3.7 Hz, 9.8 Hz, 1H), δ 1.13 (d, J = 6.4 Hz, 3H)
¹⁹F NMR (376 MHz, CDCl₃) δ -80.6 (s, 3F), δ -118.8 (dt, J = 271 Hz, 14 Hz, 1F), δ -119.7 (dt, J = 272 Hz, 14 Hz, 1F), δ -121.3 (s, 2F), δ -121.7 (s, 2F), δ -122.2 (s, 2F), δ -122.6 (s, 2F), δ -126.0 (s, 2F)

### (3) Amidite formation

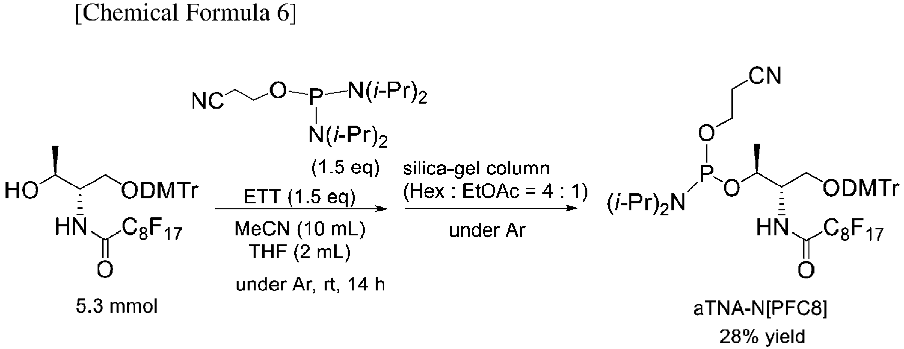

5-Ethylthiotetrazole (ETT) (1.03 g, 7.9 mmol) was added under argon to a solution obtained by dissolving 3-((bis(diisopropylamino)phosphanyl)oxy)propanenitrile (2.38 g, 7.9 mmol) in dry acetonitrile, and the DMTr-protected intermediate (4.5 g, 5.3 mmol, a solution obtained by dissolving in a mixed solvent of 2 mL of tetrahydrofuran (THF) and 10 mL of acetonitrile) was further added thereto gradually. The obtained reaction mixture was kept stirring at room temperature under argon for 14 hours. After evaporation of the solvent under reduced pressure, the obtained crude product was purified by column chromatography under argon using degassed hexane/ethyl acetate (1:4 (volume ratio)) as a mobile phase. As a result, aTNA-N[PFC] was isolated as a colorless oil (yield: 28%).

¹H NMR (400 MHz, CDCl₃) δ 7.41-7.13 (m, 9H), δ 6.84-6.79 (m, 4H), δ 4.33-4.20 (m, 1H), δ 4.14-4.07 (m, 1H), δ 3.82-3.72 (m, 6H), δ 3.63-3.43 (m, 4H), δ 3.33-3.13 (m, 2H), δ 2.57 (t, 6.4 Hz, 1H), δ 2.43-2.39 (m, 1H), δ 1.27-1.08 (m, 12H), δ 0.96 (d, J = 6.9 Hz, 3H)
¹⁹F NMR (376 MHz, CDCl₃) δ -80.6 (m, 2F), δ -118.8 (dt, J = 278 Hz, 13 Hz, 1F), δ -119.8 (dt, J = 272 Hz, 13 Hz, 1F), δ -121.3 (s, 2F), δ -121.7 (s, 2F), δ -122.1 (s, 2F), δ -122.6 (s, 2F), ³¹P NMR (162 MHz, CDCl₃), δ -149.6 (s), δ -148.5 (s)

Using the synthesized aTNA-N[PFC8], as described above, a nucleic acid (aTNA-N5[PFC8]) was synthesized in which five structures shown below (aTNA-N1 [PFC8]: in the formula, the filled circle indicates a bond) were linked to the 5'-end of control DNA (Seq. No. 1), which was natural single stranded DNA.

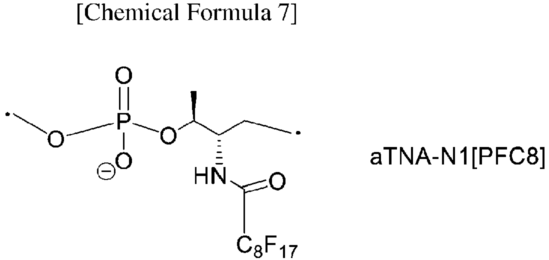

After introducing a nucleic acid (fluorescein-labeled aTNA-N5[PFC8]) in which fluorescein was bound to the 3'-end of the synthesized aTNA-N5[PFC8] into HeLa cells, flow cytometry was performed, and the number of cells into which the fluorescein-labeled nucleic acid was introduced and the amount of fluorescein-labeled nucleic acid introduced into each cell were quantified based on the fluorescence intensity of fluorescein. As a control, a nucleic acid in which fluorescein was bound to the 3'-end of the control DNA (fluorescein-labeled control DNA) was also introduced into HeLa cells in the same manner and then analyzed by flow cytometry.

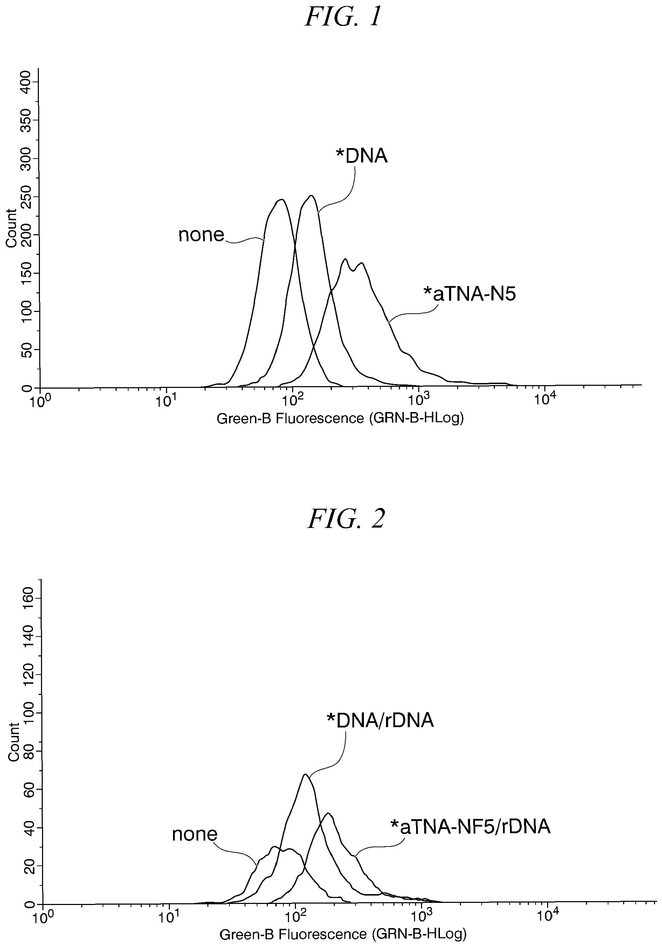

Table 1 shows the relative fluorescence intensity of cells into which each fluorescein-labeled nucleic acid was introduced ([fluorescence intensity of cells into which the fluorescein-labeled nucleic acid was introduced]/[fluorescence intensity of cells into which the fluorescein-labeled control DNA was introduced]), which was obtained by assuming that the fluorescence intensity of cells into which the fluorescein-labeled control DNA was introduced was 1. In addition, FIG. 1 shows the results of flow cytometry of cells into which the fluorescein-labeled control DNA was introduced and cells into which the fluorescein-labeled aTNA-N5[PFC8] was introduced. In the figure, "none" indicates the results of cells into which DNA was not incorporated, "DNA" indicates the results of cells into which the fluorescein-labeled control DNA was introduced, and "aTNA-N5" indicates the results of cells into which the fluorescein-labeled aTNA-N5[PFC8] was introduced.

**[Table 1]**

| Fluorescein-labeled nucleic acid | Relative fluorescence intensity of cells into which fluorescein-labeled nucleic acid was introduced |
|---|---|
| Control DNA | 1.0 |
| aTNA-N5[PFC8] | 2.52 |

As shown in Table 1, the number of cells into which the fluorescein-labeled aTNA-N5[PFC8] was introduced was nearly twice as high as that of cells into which the fluorescein-labeled control DNA was introduced. In addition, as shown in FIG. 1, many of the cells into which the fluorescein-labeled aTNA-N5[PFC8] was introduced had high fluorescence intensity per cell, and the amount of fluorescein-labeled nucleic acid introduced per cell was large. It became clear from these results that aTNA-N5[PFC8] had higher cell membrane permeability than that of the control DNA.

### [Example 2]

The fluorescein-labeled aTNA-N5[PFC8] produced in Example 1 was hybridized with a single-stranded DNA (control rDNA) composed of a base sequence (SEQ ID NO: 2) complementary to that of the control DNA to produce a fluorescein-labeled double-stranded DNA (fluorescein-labeled aTNA-N5[PFC8]/rDNA) having a repeating structure of the structure (A1) at the 5'-end and bound with fluorescein at the 3'-end. Similarly, fluorescein-labeled double-stranded DNA (fluorescein-labeled control DNA/rDNA) was produced by hybridizing fluorescein-labeled control DNA and control rDNA. These fluorescein-labeled double-stranded DNAs were introduced into HeLa cells and then analyzed by flow cytometry in the same manner as in Example 1.

Table 2 shows the relative fluorescence intensity of cells into which each fluorescein-labeled nucleic acid was introduced (assuming that the fluorescence intensity of cells into which the fluorescein-labeled control DNA/rDNA was introduced was 1). In addition, FIG. 2 shows the results of flow cytometry of cells into which each fluorescein-labeled nucleic acid was introduced. In the figure, "none" indicates the results of cells into which DNA was not incorporated, "DNA/rDNA" indicates the results of cells into which the fluorescein-labeled control DNA/rDNA was introduced, and "aTNA-NF5" indicates the results of cells into which the fluorescein-labeled aTNA-N5[PFC8]/rDNA was introduced.

**[Table 2]**

| Fluorescein-labeled nucleic acid | Relative fluorescence intensity of cells into which fluorescein-labeled nucleic acid was introduced |
|---|---|
| Control DNA/rDNA | 1.0 |
| aTNA-N5[PFC8]/rDNA | 2.3 |

As shown in Table 2 and FIG. 2, the number of cells into which the fluorescein-labeled aTNA-N5[PFC8]/rDNA was introduced was higher than the number of cells into which the fluorescein-labeled control DNA/rDNA was introduced. From these results, it was confirmed that even with double-stranded DNA, as with single-stranded DNA, the effect of improving the cell membrane permeability by the structure (A1) was exhibited.

### [Example 3]

A nucleic acid having the structure (A2) in which R⁰ was a perfluorooctyl group was synthesized.

A phosphoramidite (aTNA-C[PFC8]) having the structure (A2) in which R⁰ was a perfluorooctyl group was synthesized as follows.

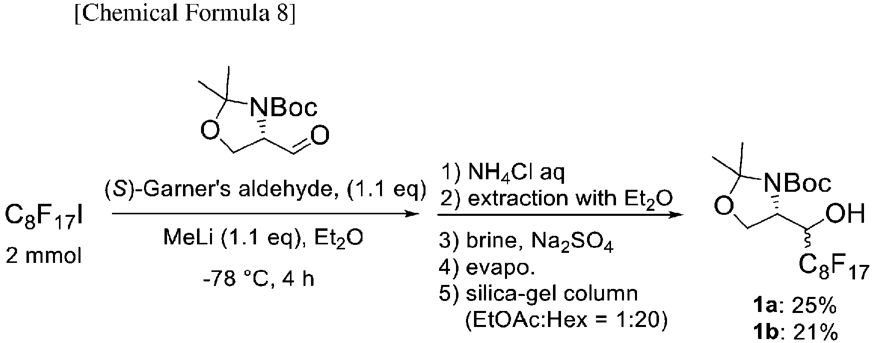

(S)-Garner's aldehyde (3.82 g, 7.0 mmol) was dissolved in dry ether (25 mL) in an oven-dried 50 mL two-neck round bottom flask, followed by the addition of C₈F₁₇I (1.76 g, 7.7 mmol), and cooled to -78°C. Subsequently, MeLi in ether (1.1 M, 7.7 mmol, 7 mL of Et₂O solution) was added dropwise to the flask over 30 minutes, and then the reaction mixture was stirred at -78°C for 4 hours. After that, the reaction was quenched with saturated NH₄Cl, and then the product was extracted with Et₂O. The organic fractions were combined, washed with brine and dried over MgSO₄, and then the solvent was removed under vacuum. The obtained crude material was purified by chromatography using a mixed solvent of EtOAc/hexane (1:20 (volume ratio)) to obtain a white solid which was a mixture of a compound 1a and a compound 1b (yield: 25% for the compound 1a and 21% for the compound 1b).

### Compound 1a:

¹H NMR (400 MHz, CDCl₃) δ 4.79 (m, 1H), 4.46 (m, 1H), 4.14 (m, 1H), 4.01 (dd, J = 10.0, 5.0 Hz, 1H), 3.90 (d, J = 9.6 Hz, 1H), 1.60 (s, 3H), 1.49 (m, 12H)
¹⁹F NMR (376 MHz, CDCl₃) δ -80.9 (s, 3F), -118.8 (d, J_{FF} = 289.0 Hz, 1F), - 121.5 - -123.7 (m, 10F), -125.3 - -127.1 (m, 2F), -127.5 (d, J_{FF} = 283.2 Hz, 1F)

### Compound 1b:

¹H NMR (400 MHz, CDCl₃) δ 4.60 (m, 1H), 4.27 (m, 2H), 4.03 (m, 1H), 3.82 (s) and 3.19 (s) (1H), 1.60 (m, 3H), 1.46 (m, 12H)
¹⁹F NMR (376 MHz, CDCl₃) δ -80.9 (s, 3F), -119.6 (d, J_{FF} = 283.2 Hz) and (d, J_{FF} = 283.2 Hz) (1F), -121.8 - -123.7 (m, 10F), -125.4 - -127.1 (m, 3F)

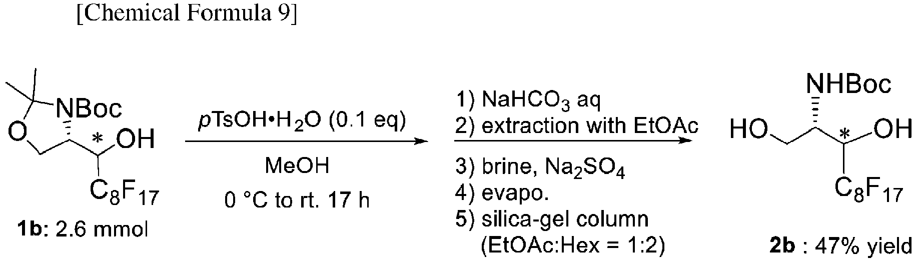

The Compound 1b (1.69 g, 2.6 mmol) was dissolved in MeOH (25 mL) in a 100 mL round bottom flask in air and cooled to 0°C. Subsequently, p-toluenesulfonic acid monohydrate (49.5 mg, 0.26 mmol) was added to the flask, and then the reaction mixture in the flask was warmed to room temperature and stirred for an additional 17 hours. After that, the reaction was quenched with saturated NaHCO₃, and then the product was extracted with EtOAc. The organic fractions were combined, washed with brine and dried over MgSO₄, and then the solvent was removed under vacuum. The obtained crude material was purified by chromatography using a mixed solvent of EtOAc/hexane (1:2 (volume ratio)) to obtain the desired compound 2b as a white solid (yield: 47%).

### Compound 2b:

¹H NMR (500 MHz, acetone-d₆) δ 6.13 (d, J = 8.6 Hz, 1H), 5.58 (d, J = 6.9 Hz, 1H), 4.52 (d, J = 24.1 Hz, 1H), 4.18 (m, 1H), 4.04 (m, 1H), 3.92 (m, 1H), 3.81 (m, 1H), 1.41 (s, 9H)
¹⁹F NMR (470 MHz, acetone-d₆) δ -81.6 (s, 3F), -118.7 (d, J_{FF} = 278.8 Hz, 1F), - 121.1 - -123.8 (m, 10F), -125.4 (d, J_{FF} = 278.8 Hz, 1F), -126.5 (d, J_{FF} = 264.1 Hz, 1F), - 127.0 (d, J_{FF} = 293.4, 1F)

A compound 2a was obtained in the same manner by using the compound 1a instead of the compound 1b.

### Compound 2a:

¹H NMR (400 MHz, acetone-d₆) δ 5.67 (dd, J = 16.9, 8.2 Hz, 1H), 4.60 (dt, J = 22.3, 5.3 Hz, 1H), 4.20 (t, J = 5.3 Hz, 1H) , 4.03 (m, 1H), 3.55 (m, 2H), .35 (s, 9H)
¹⁹F NMR (376 MHz, acetone-d₆) δ -81.5 (s, 3F), -119.3 (d, J_{FF} = 286.1 Hz, 1F), - 121.1 - -123.3 (m, 10F), -126.2 (d, J_{FF} = 300.5 Hz, 1F), -126.8 (d, J_{FF} = 283.2 Hz, 1F), - 127.1 (d, J_{FF} = 289.0 Hz, 1F)

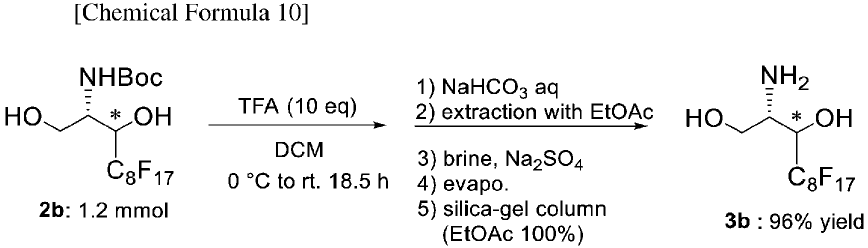

The compound 2b (746 mg, 1.2 mmol) was dissolved in dry dichloromethane (DCM) (10 mL) in a 200 mL two-neck round bottom flask and cooled to 0°C. Subsequently, trifluoroacetic acid (TFA) (0.94 mL, 12.2 mmol) was added dropwise to the flask over 10 minutes, and then the reaction mixture in the flask was warmed to room temperature and stirred for an additional 18.5 hours. After that, the reaction was quenched with saturated NaHCO₃, and then the product was extracted with EtOAc. The organic fractions were combined, washed with brine and dried over MgSO₄, and then the solvent was removed under vacuum. The obtained crude material was purified by chromatography using a mixed solvent of EtOAc/hexane (1:2 (volume ratio)) to obtain the desired compound 3b as a white solid (yield: 98%).

### Compound 3b:

¹H NMR (500 MHz, acetone-d₆) δ 5.68 (s, 1H), 4.45 (d, J = 23.5 Hz, 1H), 3.86 (t, J = 10.9 Hz, 1H), 3.78 (d, J = 8.0 Hz, 1H), 3.76 (d, J = 7.5 Hz, 1H)
¹⁹F NMR (470 MHz, acetone-d₆) δ -81.6 (s, 3F), -118.6 (d, J_{FF} = 278.8 Hz, 1F), - 121.5 - -123.9 (m, 10F), -124.9 (d, J_{FF} = 278.8 Hz, 1F), -126.3 (d, J_{FF} = 293.4 Hz, 1F), - 127.0 (d, J_{FF} = 293.4 Hz, 1F)

A compound 3a was obtained in the same manner by using the compound 2a instead of the compound 2b.

### Compound 3a:

¹H NMR (400 MHz, acetone-d₆) isomer I δ 4.25 (dd, J = 23.8, 6.9 Hz, 1H), 3.72 (m, 2H), 3.55 - 3.44 (m, 1H) isomer II δ 4.07 (dd, J = 24.9, 2.5 Hz, 1H), 3.84 (d, J = 4.1 Hz, 1H), 3.82 (d, J = 4.6 Hz, 1H), 3.35 (t, J = 6.6, 1H)
¹⁹F NMR (376 MHz, acetone-d₆) δ -81.7 (s, 3F), -120.2 (d, J_{FF} = 280.3 Hz, 1F), - 121.9 - -123.3 (m, 10F), -126.1 - -127.7 (m, 2F), -127.5 (d, J_{FF} = 283.2 Hz, 1F)

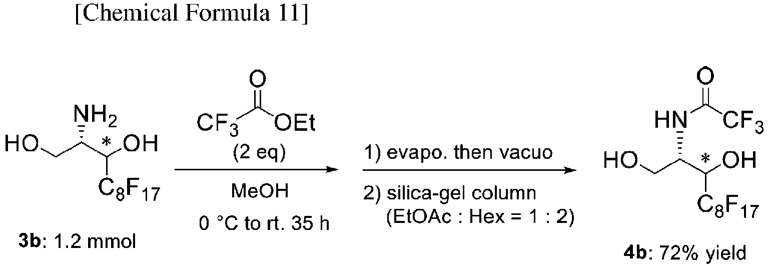

The compound 3b (598 mg, 1.2 mmol) was dissolved in dry MeOH (4 mL) in a 25 mL two-neck round bottom flask and cooled to 0°C. Subsequently, ethyl trifluoroacetate (287 µL, 340 mg, 2.4 mmol) was added dropwise to the flask over 10 minutes, and then the reaction mixture in the flask was warmed to room temperature and stirred for an additional 35 hours. The solvent was then evaporated under vacuum, and the obtained white oil was purified by chromatography using a mixed solvent of EtOAc/hexane (1:2 (volume ratio)) to obtain the desired compound 4b as a white solid (yield: 72%).

### Compound 4b:

¹H NMR (400 MHz, acetone-d₆) δ 8.50 (d, J = 8.7 Hz, 1H), 5.71 (d, J = 8.2 Hz, 1H), 4.62 (m, 1H), 4.43 (m, 1H), 4.34 (m, 1H), 3.98 (m, 1H), 3.89 (m, 1H)
¹⁹F NMR (376 MHz, acetone-d₆) δ -76.5 (s, 3F), -82.0 (s, 3F), -118.1 (d, J = 283.2 Hz, 1F), -120.9 - -124.4 (m, 10F), -125.5 (d, J = 283.2 Hz, 1F), -126.9 (d, J = 294.8 Hz, 1F), -127.5 (d, J = 289.0 Hz, 1F)

A compound 4a was obtained in the same manner by using the compound 3a instead of the compound 3b.

### Compound 4a:

¹H NMR (400 MHz, acetone-d₆) δ 8.00 (d, J = 8.7 Hz, 1H), 6.00 (s, 1H), 4.73 (dd, J = 21.3, 3.9 Hz, 1H), 4.49 (m, 2H), 3.69 (m, 2H)
¹⁹F NMR (376 MHz, acetone-d₆) δ -76.4 (s, 3F), -81.6 (s, 3F), -118.9 (d, J_{FF} = 283.2 Hz, 1F), -121.3 - -123.2 (m, 10F), -125.8 - -127.5 (m, 3F)

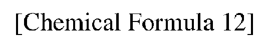

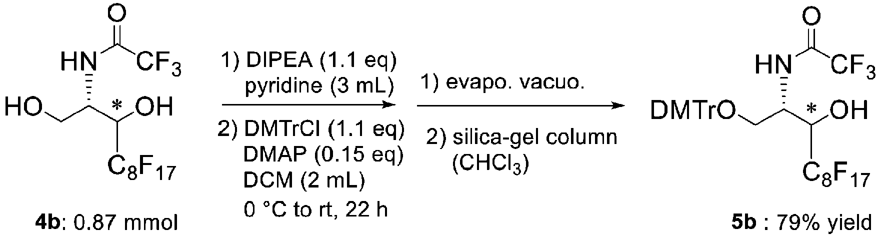

The compound 4b (526 mg, 0.87 mmol) was dissolved in dry pyridine (2 mL) in an oven-dried 25 mL two-neck round bottom flask and cooled to 0°C. Subsequently, DIPEA (167 µL, 124 mg, 0.96 mmol) was added to the flask, followed by dropwise addition of 4,4'-dimethoxytrityl chloride (325 mg, 0.96 mmol, 3 mL of dry dichloromethane solution) and 4-dimethylaminopyridine (12.2 mg, 0.1 mmol) over 10 minutes, and then the reaction mixture in the flask was warmed to room temperature and stirred for an additional 22 hours. The solvent was then evaporated under vacuum, and the obtained reaction product was purified by chromatography using CHCl₃ to obtain the desired compound 5b as a yellow oil (yield: 79%).

### Compound 5b:

¹H NMR (400 MHz, acetone-d₆) δ 8.83 (d, J = 8.7 Hz, 1H), 7.46 - 7.14 (m, 9H), 6.87 (dd, J = 8.9, 2.3 Hz, 4H), 5.83 (d, J = 8.7 Hz, 1H), 4.77 - 4.62 (m, 2H), 3.76 (s, 6H), 3.50 (m, 2H)
¹⁹F NMR (376 MHz, acetone-d₆) δ -76.2 (s, 3F), -81.5 (s, 3F), -117.4 (d, J_{FF} = 289.0 Hz, 1F), -120.5 - -123.3 (m, 10F), -125.5 (d, J_{FF} = 289.0 Hz, 1F), -126.2 (d, J_{FF} = 289.0 Hz, 1F), -127.2 (d, J_{FF} = 289.0 Hz, 1F)

A compound 5a was obtained in the same manner by using the compound 4a instead of the compound 4b.

### Compound 5a:

¹H NMR (500 MHz, acetone-d₆) δ 8.10 (s, 1H), 7.41 (d, J = 7.5 Hz, 2H) 7.29 - 7.13 (m, 7H), 6.84 (dd, J = 8.6, 1.7 Hz, 4H), 5.94 (s, 1H), 4.76 (dd, J = 20.6, 3.4 Hz, 1H), 4.70 (s, 1H), δ 3.74 (s, 6H), δ 3.34 (m, 2H)
¹⁹F NMR (376 MHz, acetone-d₆) δ -76.2 (s, 3F), -81.5 (s, 3F), -119.0 (d, J_{FF} = 283.2 Hz, 1F), -121.3 - -123.2 (m, 10F), -125.8 - -127.5 (m, 3F)

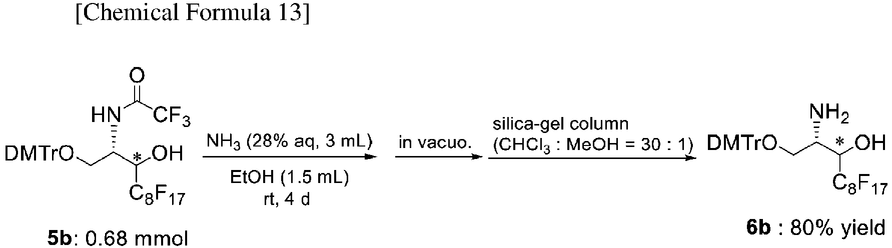

The compound 5b (623 mg, 0.68 mmol) was dissolved in ethanol (1.5 mL) in a 50 mL round bottom flask, 28% aqueous ammonia (3 mL) was added thereto, and the resulting mixture was then stirred at room temperature for 4 days. Subsequently, the solvent was evaporated from the reaction product in the flask under vacuum and subjected to chromatography using CHCl₃/MeOH (30:1 (volume ratio)) to obtain the desired compound 6b as a yellow oil (yield: 80%).

### Compound 6b:

¹H NMR (500 MHz, acetone-d₆) δ 7.49 - 7.19 (m, 9H), 6.89 (d, J = 8.0 Hz, 4H), 4.47 (dd, J = 29.8, 6.3 Hz, 1H), 4.05 (m, 1H), 3.78 (s, 6H), 3.58 - 3.48 (m, 2H)
¹⁹F NMR (376 MHz, acetone-d₆) δ -81.6 (s, 3F), -119.0 (d, J_{FF} = 294.8 Hz, 1F), - 120.9 - -124.2 (m, 10F), -125.7 - -127.7 (m, 3F)

A compound 6a was obtained in the same manner by using the compound 5a instead of the compound 5b.

### Compound 6a:

¹H NMR (500 MHz, acetone-d₆) δ 7.44 (d, J = 8.0 Hz, 2H), 7.32 - 7.20 (m, 7H), 6.87 (d, J = 9.2 Hz, 4H), 4.28 (dd, J = 23.2, 6.5 Hz, 1H), 3.86 (m, 1H), 3.78 (s, 6H), 3.40 (dd, J = 10.0, 4.2 Hz, 1H), 3.33 (dd, J = 9.7, 4.0 Hz, 1H), 2.80 (s, 2H)
¹⁹F NMR (470 MHz, acetone-d₆) δ -81.5 (s, 3F), -121.4 (d, J_{FF} = 278.8 Hz, 1F), - 122.1 - -123.9 (m, 10F), -125.9 - -127.3 (m , 3F)

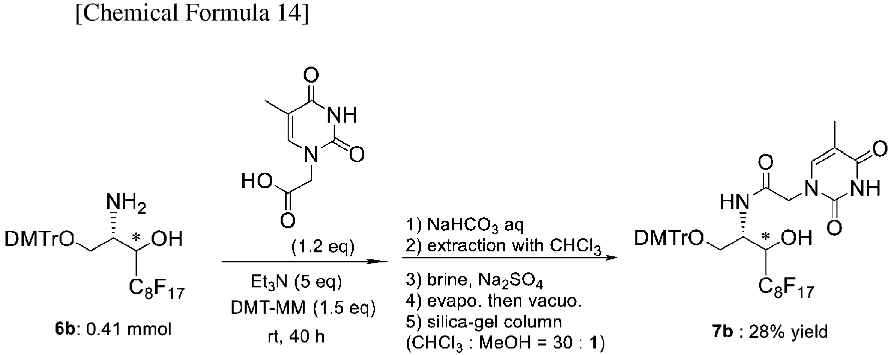

The compound 6b (332 mg, 0.41 mmol) and thymine-1-acetic acid (90 mg, 0.49 mmol) were dissolved in dry N,N-dimethylformamide (DMF) (10 mL) in a 25 mL two-neck round bottom flask. After that, dry triethylamine (285 µL, 2.04 mmol) and a dehydration condensation agent DMT-MM (CAS No: 3945-69-5) (170 mg, 0.61 mmol) were further added into the flask, and the resulting mixture was then stirred at room temperature for 40 hours. After that, the reaction was quenched with saturated NaHCO₃, and then the product was extracted with CHCl₃. The organic fractions were combined, washed with brine and dried over MgSO₄, and then the solvent was removed under vacuum. The obtained crude material was purified by chromatography using a mixed solvent of CHCl₃/MeOH (30:1 (volume ratio)) to obtain the desired compound 7b as a yellow oil (yield: 28%).

### Compound 7b:

¹H NMR (400 MHz, acetone-d₆) δ 10.2 (s, 1H), 8.02 (m, H), 7.49 - 7.20 (m, 10H), 6.89 (m, 4H), 4.59 (m, 1H), 4.58 (d, J = 16.0 Hz, 1H), 4.48 (d, J = 16.0 Hz, 1H), 3.76 (s, 6H), 3.56 (m, 2H), 3.42 (m, 1H), 1.80 (d, J = 0.9 Hz, 3H)
¹⁹F NMR (376 MHz, acetone-d₆) δ -81.5 (s, 3F), -117.8 (d, J_{FF} = 294.8 Hz, 1F), - 120.9 - -123.3 (m, 10F), -125.1 (d, J_{FF} = 283.2 Hz, 1F), -126.2 (d, J_{FF} = 294.8 Hz, 1F), - 127.1 (d, J_{FF} = 289.0 Hz, 1F)
LRMS(ESI-TOF): calcd for C₃₉H₃₂F₁₇N₃NaO₇[M+Na]⁺: 1000.19, found: 999.87

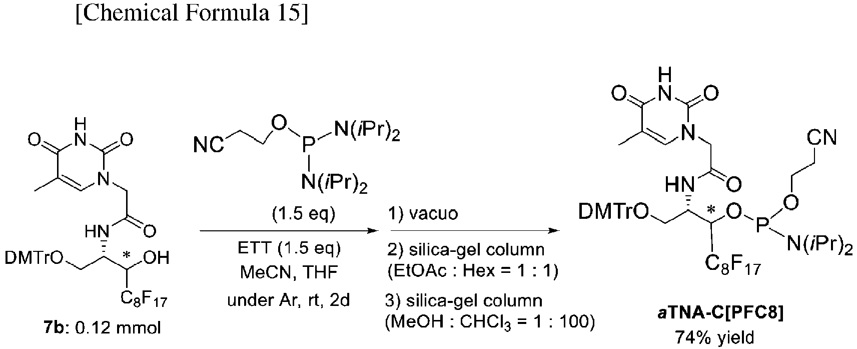

3-((Bis(diisopropylamino)phosphanyl)oxy)propanenitrile (57 mg, 0.19 mmol) was dissolved in dry acetonitrile. ETT (57 mg, 0.19 mmol) was added to this solution under argon, and then a compound 7b (123 mg, 0.13 mmol, a solution obtained by dissolving in a mixed solvent of 0.6 mL of THF and 0.6 mL of acetonitrile) was further added gradually, and the resulting mixture was then stirred at room temperature for 2 days. After that, the solvent was evaporated under vacuum, and the obtained crude product was then purified by chromatography using EtOAc/hexane (1:1 (volume ratio)) under argon and chromatography using MeOH/HCl₃ (1:100 (volume ratio)) under air to obtain aTNA-C[PFC8] as a yellow oil (yield: 74%).

¹H NMR (500 MHz, acetone-d₆) δ 10.2 (s, 1H), 7.95 (d, J = 8.0 Hz, 1H), 7.49 - 7.21 (m, 10H), 6.91 - 6.87 (m, 4H), 4.84 - 4.47 (m, 3H), 4.00 - 3.91 (m, 1H), 3.78 (s, 6H), 3.60 - 3.31 (m, 7H), 2.93 - 2.67 (m, 2H), 1.81 (m, 3H), 1.31 - 1.00 (m, 12H)
¹⁹F NMR (470 MHz, acetone-d₆) isomer I: δ -81.5 (s, 3F), -116.6 (d, J_{FF} = 278.8 Hz, 1F), -120.0 (d, JFF = 249.4 Hz, 1F), -120.9 - -123.8 (m, 10F), -126.3 (d, JFF = 308.1 Hz, 1F), δ -127.0 (d, JFF = 293.4 Hz, 1F) isomer II: δ -81.5 (s, 3F), -117.8 (d, JFF = 293.4 Hz, 1F), -120.9 - -123.8 (m, 10F), -125.1 (d, JFF = 278.8 Hz, 1F), -126.3 (d, JFF = 308.1 Hz, 1F), -127.0 (d, JFF = 293.4 Hz, 1F)
³¹P NMR (202 MHz, Acetone-d₆), isomer I: δ -154.2 (s), isomer II: δ -152.1 (s)
LRMS(ESI-TOF): calcd for C₄₈H₄₉F₁₇N₅NaO₈P[M+Na]⁺: 1200.29, found: 1199.86

Using the synthesized aTNA-C[PFC8], as described above, nucleic acids (aTNA-C2[PFC8], aTNA-C5[PFC8]) were synthesized in which two or five structures shown below (aTNA-C1[PFC8]: in the formula, the filled circle indicates a bond) were linked to the 5'-end of control DNA (Seq. No. 1), which was natural DNA.

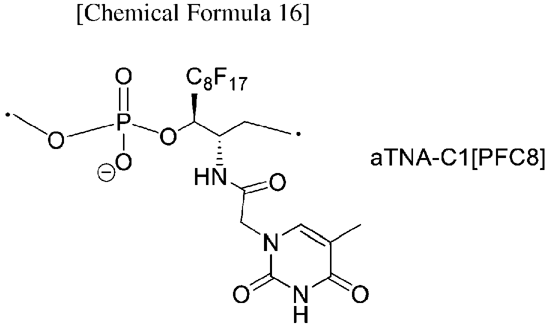

After respectively introducing nucleic acids (fluorescein-labeled aTNA-C2[PFC8] and fluorescein-labeled aTNA-C5[PFC8]) in which fluorescein was bound to the 3'-ends of the synthesized aTNA-C2[PFC8] and aTNA-C5[PFC8] into HeLa cells, flow cytometry was performed, and the number of cells into which the fluorescein-labeled nucleic acid was introduced and the amount of fluorescein-labeled nucleic acid introduced into each cell were quantified based on the fluorescence intensity of fluorescein. As a control, a fluorescein-labeled control DNA was also introduced into HeLa cells in the same manner and then analyzed by flow cytometry.

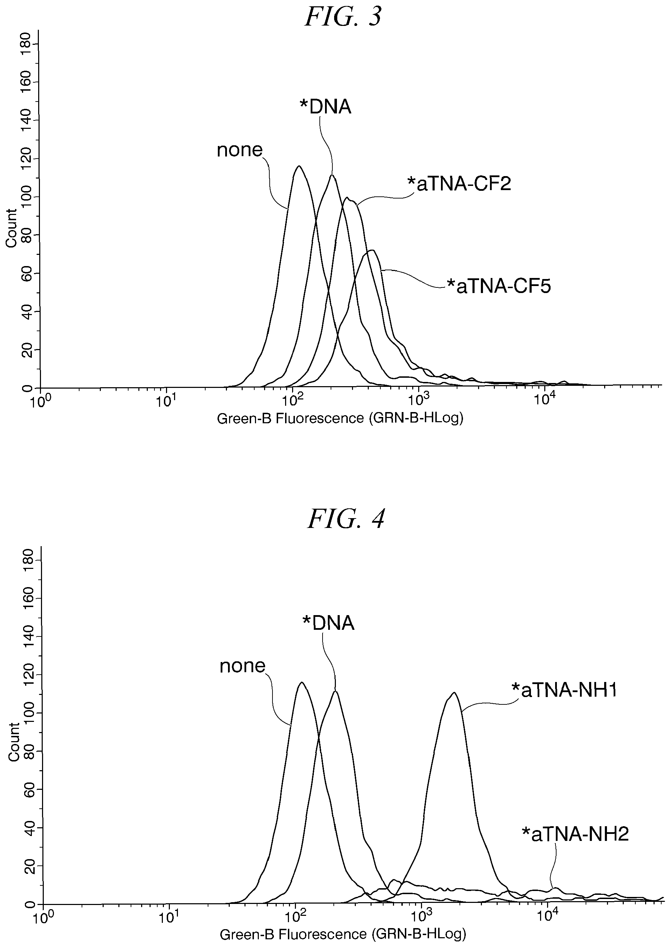

Table 3 shows the relative fluorescence intensity of cells into which each fluorescein-labeled nucleic acid was introduced (assuming that the fluorescence intensity of cells into which the fluorescein-labeled control DNA was introduced was 1). In addition, FIG. 3 shows the results of flow cytometry of cells into which each fluorescein-labeled nucleic acid was introduced. In the figure, "none" indicates the results of cells into which DNA was not incorporated, "DNA" indicates the results of cells into which the fluorescein-labeled control DNA was introduced, "aTNA-C2" indicates the results of cells into which the fluorescein-labeled aTNA-C2[PFC8] was introduced, and "aTNA-C5" indicates the results of cells into which the fluorescein-labeled aTNA-C5[PFC8] was introduced.

**[Table 3]**

| Fluorescein-labeled nucleic acid | Relative fluorescence intensity of cells into which fluorescein-labeled nucleic acid was introduced |
|---|---|
| Control DNA | 1.0 |
| aTNA-C2[PFC8] | 1.7 |
| aTNA-C5[PFC8] | 2.6 |

As shown in Table 3 and FIG. 3, the number of cells into which the fluorescein-labeled aTNA-C2[PFC8] was introduced and the number of cells into which the fluorescein-labeled aTNA-C5[PFC8] was introduced were higher than that of cells into which the fluorescein-labeled control DNA was introduced, and these were found to have higher cell membrane permeability than that of the control DNA. In addition, it was found that the fluorescein-labeled aTNA-C5[PFC8] was introduced into a larger number of cells and had higher cell permeability, as compared with the fluorescein-labeled aTNA-C2[PFC8].

### [Example 4]

A nucleic acid having the structure (A1) in which R⁰ was a heptadecyl group was synthesized, and the cell membrane permeability was examined.

A phosphoramidite (aTNA-N[HC17]) having the structure (A1) in which R⁰ was a heptadecyl group was synthesized as follows.

### (1) Amidite condensation

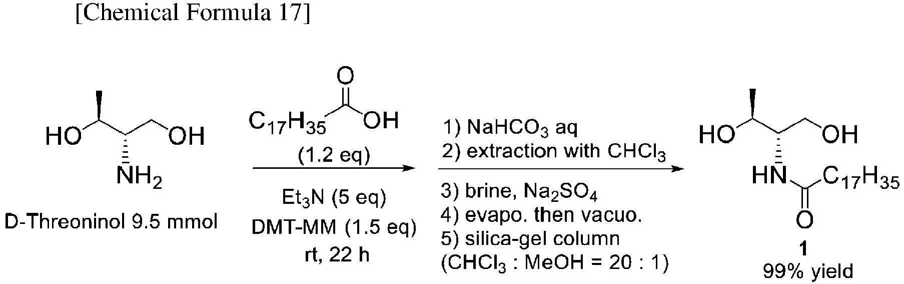

D-threoninol (1 g, 9.5 mmol) and stearic acid (3.24 g, 11.4 mmol) were placed and dissolved in a 200 mL two-neck round bottom flask containing dry DMF (10 mL), dry triethylamine (6.62 mL, 47.5 mmol) and DMT-MM (3.94 g, 14.3 mmol). After stirring for 22 hours at room temperature, the reaction was quenched with saturated NaHCO₃, and the product was extracted with CHCl₃. The organic fractions were combined, washed with brine and dried over MgSO₄, and then the solvent was removed under vacuum. The obtained crude material was purified by chromatography using a mixed solvent of CHCl₃/MeOH (20:1 (volume ratio)) to obtain the desired intermediate 1 as a yellow oil (yield: 99%).

¹H NMR (400 MHz, CDCl₃) δ 6.20 (d, J = 6.9 Hz, 1H), δ 4.21-4.17 (m, 1H), δ 3.87-3.80 (m, 3H), δ 2.76-2.62 (m, 2H), δ 2.25 (t, 2H), δ 1.37-1.25 (m, 33H), δ 0.93-0.82 (m, 3H)

### (2) DMTr protection

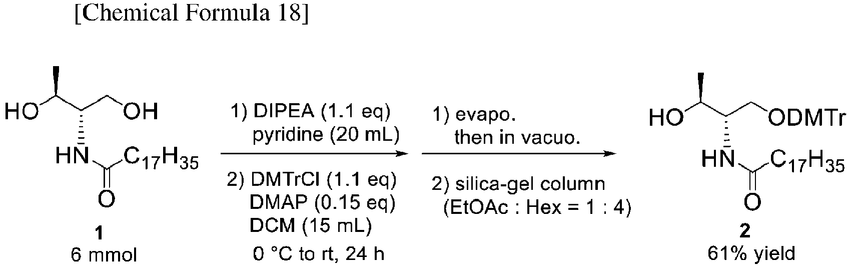

The intermediate 1 (2.23 g, 6.0 mmol) was dissolved in dry pyridine (20 mL) in an oven-dried 200 mL two-neck round bottom flask and cooled to 0°C. Subsequently, diisopropylethylamine (1.15 mL, 6.6 mmol) was added to this mixture, and 4,4'-dimethoxytrityl chloride (2.24 g, 6.6 mmol) and 4-dimethylaminopyridine (80.6 mg, 0.66 mmol) dissolved in 15 mL of dry dichloromethane were further added dropwise thereto over 10 minutes. The resulting mixture was warmed to room temperature and then stirred for 24 hours. The solvent was then removed under vacuum. The obtained crude material was purified by chromatography using a mixed solvent of EtOAc/hexane (1:4 (volume ratio)) to obtain a yellow oil which was a DMTr protected intermediate 2 (yield: 61%).

¹H NMR (400 MHz, CDCl₃) δ 7.37-7.19 (m, 9H), δ 6.82 (d, J = 9.2 Hz, 4H), δ 6.07 (d, J = 8.7 Hz, 1H), δ 4.09-4.04 (m, 1H), δ 3.94-3.90 (m, 1H), δ 3.78 (s, 6H), δ 3.42 (dd, J = 4.1 Hz, 9.6 Hz, 1H), δ 3.26 (dd, J = 3.2 Hz, 9.6 Hz, 1H), δ 3.10 (d, J = 2.3 Hz, 1H), δ 2.21 (t, J = 7.5 Hz, 2H), δ 1.30-1.20 (m, 30H), δ 1.11 (d, 3H), δ 0.87 (t, J = 6.5 Hz, 3H)

### (3) Amidite formation

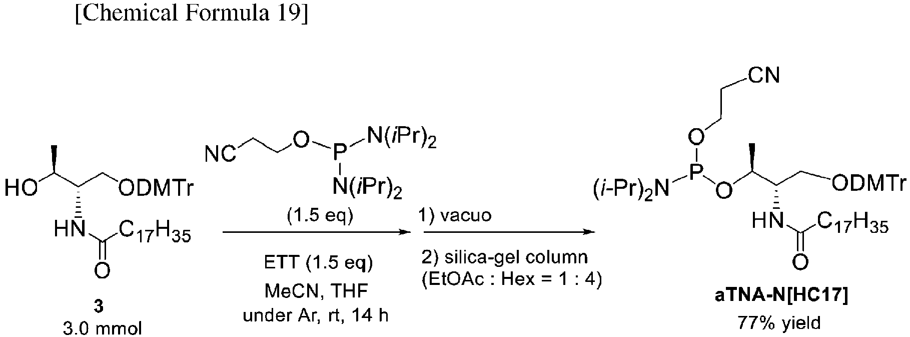

ETT (424 mg, 3.26 mmol) was added under argon to a solution obtained by dissolving 3-((bis(diisopropylamino)phosphanyl)oxy)propanenitrile (983 mg, 3.26 mmol) in dry acetonitrile, and the DMTr protected intermediate 3 (2.0 g, 2.97 mmol, a solution obtained by dissolving in a mixed solvent of 5 mL of THF and 10 mL of acetonitrile) was further added thereto gradually. The obtained reaction mixture was kept stirring at room temperature under argon for 14 hours. After evaporation of the solvent under reduced pressure, the obtained crude product was purified by column chromatography under argon using hexane/ethyl acetate (1:4 (volume ratio)) as a mobile phase. As a result, aTNA-N[HC17] was isolated as a yellow oil (yield: 77%).

### ¹H NMR (400MHz, CDCl₃)

isomer I: δ 7.41-7.18 (m, 9H), δ 6.82-6.80 (m, 4H), δ 5.75 (d, 1H), δ 4.38-4.30 (m, 1H), δ 4.22-4.16 (m, 1H) δ 3.77 (m, 6H) δ 3.61-3.40 (m, 4H) δ 3.22-3.09 (m, 2H), δ 2.43-2.26 (m, 2H), δ 2.19-2.12 (m, 2H), δ 1.28-1.11 (m, 45H), δ 0.86 (d, J = 6.9 Hz, 3H)
isomer II: δ 7.39 (d, 2H), δ 7.29-7.16 (m, 7H), δ 6.80 (dd, J = 1.4 Hz, 8.70 Hz, 4H), δ 5.59 (d, J = 8.7 Hz, 1H), δ 4.25-4.14 (m, 2H), δ 3.77 (m, 6H), δ 3.68-3.64 (m, 1H), δ 3.50-3.44 (m, 2H), δ 3.21 (dd, J = 6.4 Hz, 9.2 Hz, 1H), δ 3.21 (dd, J = 6.9 Hz, 9.2 Hz, 1H), δ 2.57 (t, 2H), δ 2.17-2.14 (m, 2H), δ 1.30-1.19 (m, 33H), δ 1.10 (d, J = 6.9 Hz, 6H), δ 0.97 (d, J = 6.9 Hz, 6H), δ 0.86 (d, J = 6.6 Hz, 3H)

Using the synthesized aTNA-N[HC17], as described above, nucleic acids (aTNA-N1 [HC17] and aTNA-N2[HC17]) were synthesized in which one or two structures shown below (aTNA-N1[HC17]: in the formula, the filled circle indicates a bond) were linked to the 5'-end of control DNA (Seq. No. 1), which was natural DNA.

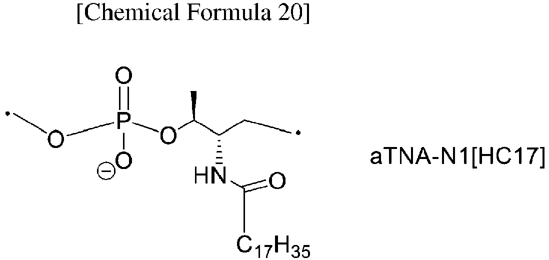

Nucleic acids (fluorescein-labeled aTNA-N1 [HC17]] and fluorescein-labeled aTNA-N2[HC17]] in which fluorescein was bound to the 3'-ends of the synthesized aTNA-N1[HC17] and aTNA-N2[HC17] were introduced into HeLa cells. More specifically, HeLa cells seeded in a 96-well plate so as to achieve a density of 10⁴ cells per well were incubated for 4 hours by changing to DMEM medium (1 mL) containing the fluorescein-labeled nucleic acid (2.5 µM). Thereafter, flow cytometry was performed in the same manner as described above, and the amount of fluorescein-labeled aTNA-N1[HC17] and the like introduced into the cells was quantified based on the fluorescence intensity of fluorescein. As a control, a nucleic acid in which fluorescein was bound to the 3'-end of the control DNA (fluorescein-labeled control DNA) was introduced into HeLa cells in the same manner, followed by flow cytometry to determine the amount of nucleic acid introduced into the cells.

Table 3 shows the relative fluorescence intensity of cells into which the fluorescein-labeled aTNA-N1[HC17] was introduced and the relative fluorescence intensity of cells into which the fluorescein-labeled aTNA-N2[HC17] was introduced (assuming that the fluorescence intensity of cells into which the fluorescein-labeled control DNA was introduced was 1). In addition, FIG. 3 shows the results of flow cytometry of cells into which the fluorescein-labeled control DNA was introduced and cells into which the fluorescein-labeled nucleic acid was introduced. In the figure, "none" indicates the results of cells into which DNA was not incorporated, "DNA" indicates the results of cells into which the fluorescein-labeled control DNA was introduced, and "aTNA-N5" indicates the results of cells into which the fluorescein-labeled aTNA-N5[PFC8] was introduced. "aTNA-NH1" indicates the results of cells into which the fluorescein-labeled aTNA-N1[HC17] was introduced, and "aTNA-NH2" indicates the results of cells into which the fluorescein-labeled aTNA-N2[HC17] was introduced.

**[Table 4]**

| Fluorescein-labeled nucleic acid | Relative fluorescence intensity of cells into which fluorescein-labeled nucleic acid was introduced |
|---|---|
| Control DNA | 1.0 |
| aTNA-N 1 [HC 17] | 2.3 |
| aTNA-N2[HC17] | 23.7 |

As shown in Table 4 and FIG. 4, the number of cells into which the fluorescein-labeled aTNA-N1[HC17] was introduced and the number of cells into which the fluorescein-labeled aTNA-N2[HC17] was introduced were higher than that of cells into which the fluorescein-labeled control DNA was introduced. In addition, the number of cells into which the fluorescein-labeled aTNA-N2[HC17] was introduced was higher than the number of cells into which the fluorescein-labeled aTNA-N1[HC17] was introduced. It became clear from these results that both aTNA-N1 [HC17] and aTNA-N2[HC17] had higher cell membrane permeability than that of the control DNA, and that the greater the number of repetitions of the structure (A1) introduced into the nucleic acid, the easier it was to be introduced into cells.

### [Example 5]

The fluorescein-labeled aTNA-N1 [HC17] produced in Example 4 was hybridized with control rDNA to produce a fluorescein-labeled double-stranded DNA (fluorescein-labeled aTNA-N1[HC17]/rDNA) having a repeating structure of the structure (A1) at the 5'-end and bound with fluorescein at the 3'-end. Similarly, the fluorescein-labeled aTNA-N2[HC17] produced in Example 4 was hybridized with control rDNA to produce a fluorescein-labeled double-stranded DNA (fluorescein-labeled aTNA-N2[HC17]/rDNA). These fluorescein-labeled double-stranded DNAs and the fluorescein-labeled control DNA/rDNA used in Example 2 were introduced into HeLa cells and then analyzed by flow cytometry in the same manner as in Example 1.

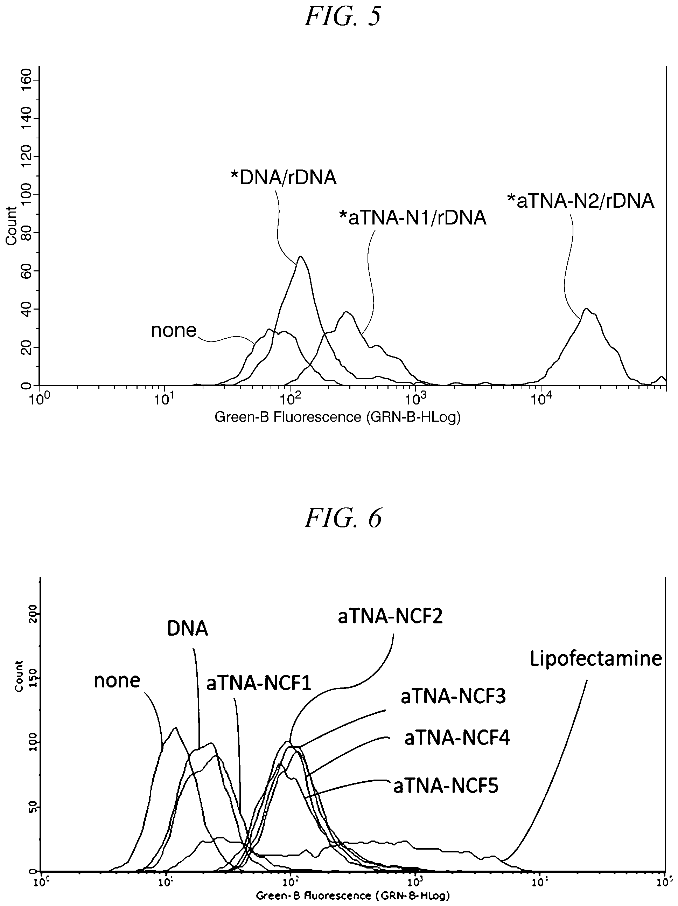

Table 5 shows the relative fluorescence intensity of cells into which each fluorescein-labeled nucleic acid was introduced (assuming that the fluorescence intensity of cells into which the fluorescein-labeled control DNA/rDNA was introduced was 1). In addition, FIG. 5 shows the results of flow cytometry of cells into which each fluorescein-labeled nucleic acid was introduced. In the figure, "none" indicates the results of cells into which DNA was not incorporated, "DNA/rDNA" indicates the results of cells into which the fluorescein-labeled control DNA/rDNA was introduced, "aTNA-N1/rDNA" indicates the results of cells into which the fluorescein-labeled aTNA-N1[HC17]/rDNA was introduced, and "aTNA-N2/rDNA" indicates the results of cells into which the fluorescein-labeled aTNA-N2[HC17]/rDNA was introduced.

**[Table 5]**

| Fluorescein-labeled nucleic acid | Relative fluorescence intensity of cells into which fluorescein-labeled nucleic acid was introduced |
|---|---|
| Control DNA/rDNA | 1.0 |
| aTNA-N1[HC17]/rDNA | 2.8 |
| aTNA-N2 [HC 17] /rDNA | 175.0 |

As shown in Table 5 and FIG. 5, the number of cells into which the fluorescein-labeled aTNA-N1 [HC17]/rDNA was introduced and the number of cells into which the fluorescein-labeled aTNA-N2[HC17]/rDNA was introduced were higher than the number of cells into which the fluorescein-labeled control DNA/rDNA was introduced. From these results, it was confirmed that even with double-stranded DNA, as with single-stranded DNA, the effect of improving the cell membrane permeability by the structure (A1) was exhibited.

### [Example 6]

A nucleic acid having the structure (A1) in which R⁰ was a group represented by the general formula (f-1), where n1 was 2, and Rf^{P} was a perfluorohexyl group, was synthesized.

A phosphoramidite (aTNA-N[FC8]) having the structure (A1) in which R⁰ was a group represented by the general formula (f-1), where n1 was 2, and Rf^{P} was a perfluorohexyl group, was synthesized as follows.

### (1) Amidite condensation

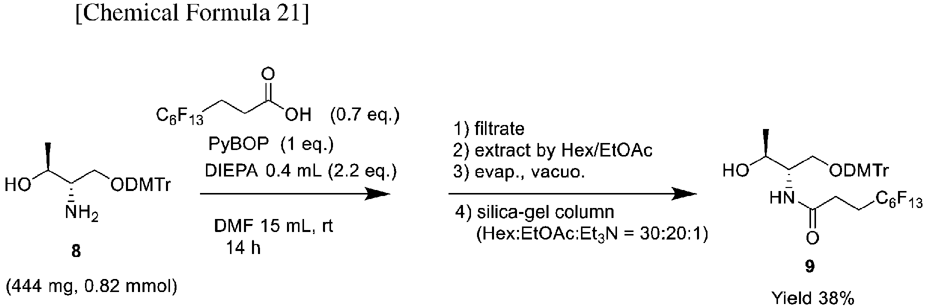

The compound 8 (0.44 g, 0.82 mmol) dissolved in 10 mL of DMF, PyBOP (0.43 g, 0.82 mmol) and DIPEA (0.4 mL, 2.2 equivalents) were dissolved in 15 mL of DMF under argon, and 4,4,5,5,6,6,7,7,8,8,9,9,9-tridecafluorononanoic acid (0.23 g, 0.59 mmol, 0.7 equivalents) was added thereto to prepare a reaction solution. The obtained reaction solution was stirred at room temperature for 14 hours. The reaction mixture was then quenched with water (45 mL) and extracted twice with hexane/ethyl acetate (4:1 (volume ratio)). The organic fractions were combined, washed with water and dried over Na₂SO₄, followed by filtration, and the solvent was removed under vacuum. The obtained crude product was purified by silica gel column chromatography (hexane: ethyl acetate: Et₃N = 30: 20: 1 (volume ratio)) to obtain a compound 9 (0.17 g, 0.22 mmol, yield: 38%).

### Compound 9:

¹H NMR (400 MHz, CDCl₃) δ 7.39-7.21 (m, 9H), 6.84 (d, J = 5.9 Hz, 4H), 6.06 (d, J = 9.1 Hz, 1H), 4.13-4.10 (m, 1H), 3.92-3.90 (m, 1H), 3.78 (s, 6H), 3.41 (dd, J = 11.2, 5.7 Hz, 1H), 3.33 (dd, J = 9.6, 3.2 Hz, 1H), 2.90 (s, 1H), 2.53-2.44 (m, 4H), 1.12 (d, J = 6.4 Hz, 3H).
¹⁹F NMR (376 MHz, CDCl₃) δ -80.7 (s, 3F), -114.4 (s, 2F), -121.8 (s, 2F), - 122.8 (s, 2F), -123.4 (s, 2F), - 126.0 (s, 2F).

### (2) DMTr protection

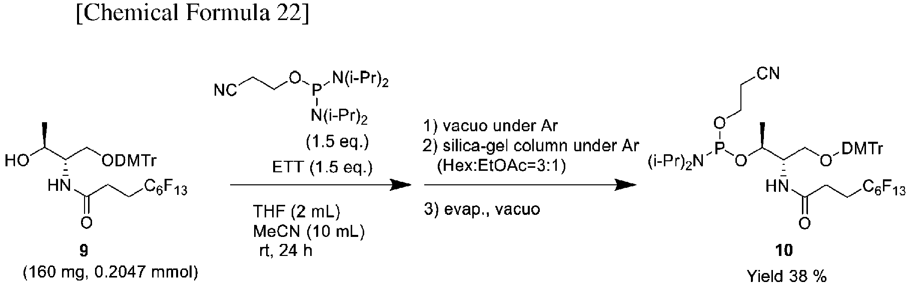

The compound 9 (160 mg, 0.20 mmol) dissolved in a mixed solvent of THF/acetonitrile (2 mL/2 mL) was added dropwise to a solution obtained by dissolving 2-cyanoethyl N,N,N',N'-tetraisopropylphosphordiamidite (93 mg, 0.31 mmol) and ETT (40 mg, 0.31 mmol) in dry acetonitrile (8 mL) under argon. After stirring the reaction mixture at room temperature for 24 hours, the solvent was evaporated under reduced pressure. The obtained crude product was purified by silica gel column chromatography under argon using degassed hexane/ethyl acetate (3:1 (volume ratio)) as a mobile phase. As a result, a compound 10 (DMTr protected aTNA-N[FC8]) was isolated as a colorless oil (yield: 38%).

### Compound 10:

¹H NMR (400 MHz, CDCl₃) δ 7.41-7.20 (m, 9H), 6.82-6.79 (m, 4H), 5.94 (d, 9.1 Hz, 1H), 4.37-4.32 (m, 1H), 4.20-4.14 (m, 1H), 3.77 (s, 6H), 3.55-3.45 (m, 4H), 3.25-3.12 (m, 2H), 2.59-2.31 (m, 6H), 1.24-0.97 (m, 15H).
¹⁹F NMR (376 MHz, CDCl₃) δ -80.7 (s, 3F), -114.5 (s, 2F), -121.8 (s, 2F), - 122.8 (s, 2F), -123.4 (s, 2F), - 126.0 (s, 2F).

Using the synthesized aTNA-N[FC8], as described above, nucleic acids (from aTNA-N1 [FC8] to aTNA-N5[FC8]) were synthesized in which one to five structures shown below (aTNA-N1[FC8]: in the formula, the filled circle indicates a bond) were linked to the 5'-end of control DNA (Seq. No. 1), which was natural DNA.

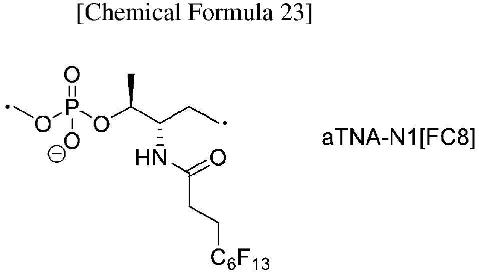

Nucleic acids (from fluorescein-labeled aTNA-N 1 [FC8] to fluorescein-labeled aTNA-N5[FC8]) in which fluorescein was bound to the 3'-ends of the synthesized aTNA-N1[FC8] to aTNA-N5[FC8] were introduced into HeLa cells. More specifically, HeLa cells seeded in a 96-well plate so as to achieve a density of 10⁴ cells per well were incubated for 4 hours by changing to DMEM medium (100 µL) containing the fluorescein-labeled nucleic acid (2.0 µM). Thereafter, flow cytometry was performed in the same manner as described above, and the amount of fluorescein-labeled aTNA-N1 [FC8] and the like introduced into the cells was quantified based on the fluorescence intensity of fluorescein. As a control, a nucleic acid in which fluorescein was bound to the 3'-end of the control DNA (fluorescein-labeled control DNA) was introduced into HeLa cells in the same manner, followed by flow cytometry to determine the amount of nucleic acid introduced into the cells. In addition, for comparison, HeLa cells were incubated for 4 hours by changing to a 10-fold dilution (100 µM) of a solution prepared with Lipofectamine using DMEM medium so that the concentration of nucleic acid (fluorescein-labeled control DNA) was 10 µM. Thereafter, flow cytometry was performed in the same manner as described above, and the amount of nucleic acid introduced into the cells was quantified based on the fluorescence intensity of fluorescein.

Table 6 shows the results from the relative fluorescence intensity of cells into which the fluorescein-labeled aTNA-N1 [FC8] was introduced to the relative fluorescence intensity of cells into which the fluorescein-labeled aTNA-N5[FC8] was introduced (assuming that the fluorescence intensity of cells into which the fluorescein-labeled control DNA was introduced was 1). In addition, FIG. 6 shows the results of flow cytometry of cells into which the fluorescein-labeled control DNA was introduced and cells into which the fluorescein-labeled nucleic acid was introduced. In the figure, "none" indicates the results of cells into which DNA was not incorporated, "DNA" indicates the results of cells into which the fluorescein-labeled control DNA was introduced, and "Lipofectamine" indicates the results of cells into which nucleic acid was introduced using Lipofectamine. "aTNA-NCF1" indicates the results of cells into which the fluorescein-labeled aTNA-N1 [FC8] was introduced, "aTNA-NCF2" indicates the results of cells into which the fluorescein-labeled aTNA-N2[FC8] was introduced, "aTNA-NCF3" indicates the results of cells into which the fluorescein-labeled aTNA-N3[FC8] was introduced, "aTNA-NCF4" indicates the results of cells into which the fluorescein-labeled aTNA-N4[FC8] was introduced, and "aTNA-NCF5" indicates the results of cells into which the fluorescein-labeled aTNA-N5[FC8] was introduced.

**[Table 6]**

| Fluorescein-labeled nucleic acid | Relative fluorescence intensity of cells into which fluorescein-labeled nucleic acid was introduced |
|---|---|
| Control DNA | 1.0 |
| aTNA-N1[FC8] | 1.3 |
| aTNA-N2[FC8] | 6.2 |
| aTNA-N3[FC8] | 6.8 |
| aTNA-N4[FC8] | 7.4 |
| aTNA-N5[FC8] | 6.0 |
| Lipofectamine | 19.6 |

As shown in Table 6 and FIG. 6, the number of cells, from those into which the fluorescein-labeled aTNA-N1 [FC8] was introduced to those into which the fluorescein-labeled aTNA-N5[FC8] was introduced, was higher than that of cells into which the fluorescein-labeled control DNA was introduced. In addition, the number of cells into which the fluorescein-labeled aTNA-N4[FC8] was introduced was higher than the number of cells into which the fluorescein-labeled aTNA-N 1 [FC8] was introduced. It became clear from these results that all of aTNA-N1 [FC8] to aTNA-N5[FC8] were inferior to Lipofectamine but had higher cell membrane permeability than that of the control DNA, and that the greater the number of repetitions of the structure (A1) introduced into the nucleic acid, the more likely it was to be introduced into cells.

### [Industrial Applicability]

The present invention provides an aTNA-type nucleic acid containing a highly hydrophobic C₁₋₃₀ alkyl group which may be substituted with a fluorine atom. Since the nucleic acid according to the present invention has excellent cell membrane permeability, it is expected to be used in the pharmaceutical field, for example, as a physiologically active substance such as a carrier for introducing a medicinal ingredient into target cells.

[Sequence Listing]

## Claims

1. A nucleic acid comprising a structure represented by: the following general formula (A1) or (A2), 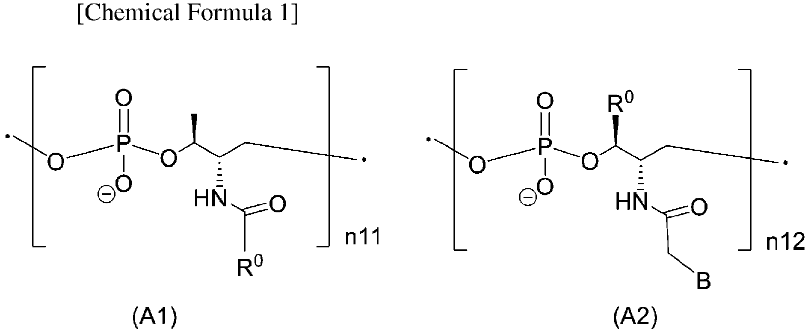 [wherein R⁰ is an alkyl group having 1 to 30 carbon atoms that is substituted with one or more fluorine atoms, a group having 1 to 5 etheric oxygen atoms between carbon atoms of an alkyl group having 2 to 30 carbon atoms that is substituted with one or more fluorine atoms, an alkyl group having 10 to 30 carbon atoms that is not substituted with a fluorine atom, or a group having 1 to 5 etheric oxygen atoms between carbon atoms of an alkyl group having 10 to 30 carbon atoms that is not substituted with a fluorine atom; n11 and n12 each independently represent an integer of 1 or more; B is a nucleobase; and a filled circle indicates a bond].

2. The nucleic acid according to Claim 1, wherein said R⁰ is an alkyl group having 1 to 30 carbon atoms that is substituted with at least two fluorine atoms.

3. The nucleic acid according to Claim 2,
wherein said R⁰ is a perfluoroalkyl group having 1 to 10 carbon atoms, or a group having 1 to 5 etheric oxygen atoms between carbon atoms of a perfluoroalkyl group having 1 to 10 carbon atoms.

4. The nucleic acid according to Claim 1,
wherein said R⁰ is an alkyl group having 10 to 30 carbon atoms that is not substituted with a fluorine atom, or a group having 1 to 5 etheric oxygen atoms between carbon atoms of an alkyl group having 10 to 30 carbon atoms that is not substituted with a fluorine atom.

5. The nucleic acid according to any one of Claims 1 to 4, wherein n11 or n12 is 5 or more.

6. The nucleic acid according to any one of Claims 1 to 5, which is cell membrane permeable.

7. A cell membrane permeabilizing agent comprising the nucleic acid according to any one of Claims 1 to 6 as an active ingredient.

8. A nucleic acid drug comprising the nucleic acid according to any one of Claims 1 to 6 as an active ingredient.
